# EUROPEAN PATENT SPECIFICATION

(11) **EP 0 707 579 B1**
(45) Date of publication and mention of the grant of the patent: **20.01.1999**
(21) Application number: 94919752.9
(22) Date of filing: 04.07.1994
(51) Int. Cl.: C07D 401/04, A61K 31/445

(54) **PIPERIDINYL SUBSTITUTED METHANOANTHRACENES AS D1/D2-ANTAGONISTS AND 5HT2-SEROTANIN-ANTAGONISTS**
PIPERIDINYL-SUBSTITUIERTE METHANOANTHRACENE ALS D1/D2-ANTAGONISTEN UND 5HT2-SEROTONIN-ANTAGONISTEN
METHANOANTHRACENES SUBSTITUES DE PIPERIDINYLE EN TANT QU'ANTAGONISTES DE D1/D2 ET EN TANT QU'ANTAGONISTES DE SEROTONINE DE 5HT2

(30) Priority: 09.07.1993 GB 9314250
(43) Date of publication of application: 24.04.1996
(73) Proprietor: ZENECA LIMITED, London W1Y 6LN (GB)
(72) Inventor: KLIMAS, Michael, Thaddeus, Exton, PA 19341 (US); TERPKO, Marc, Ornal, Wilmington, DE 19810 (US)
(74) Representative: Bill, Kevin
(86) International application number: GB9401439
(87) International publication number: WO9501974

(56) References cited:
- EP-A- 0 533 344

## Description

### BACKGROUND OF THE INVENTION

The present invention relates to novel piperidinyl compounds which contain a chiral sulfoxide moiety and processes related to their synthesis and production. The compounds and the pharmaceutical compositions containing the active ingredient are useful as dopamine antagonists and are particularly useful as antipsychotic agents in humans. The compounds may also be useful in other neurological and psychiatric disorders including schizophrenia. Certain methanoanthracene compounds containing racemic sulfoxides useful as D2 dopamine receptor antagonists have been disclosed. See EPA 0 533 344 A1. The present invention, on the other hand, relates to methanoanthracenyl compounds that are essentially or substantially pure chiral sulfoxide enantiomers or diastereomers and pharmaceutical compositions containing said active ingredients wherein the compounds are useful as D1 and D2 dopamine antagonists as well as 5HT2 serotonin antagonists. The claimed compounds have an improved, balanced and more favorable D1/D2 dopamine antagonist ratio which may be predictive of an improved clinical profile in humans. The present compounds are also serotonin 5HT2 antagonists. The present invention also relates to novel syntheses of chiral methanoanthracenyl sulfoxides wherein the process utilizes several oxidation methods to selectively oxidize a methanoanthracenyl sulfide to an enantiomerically enriched sulfoxide mixture which can be further purified to a pure or substantially pure methanoanthrancenyl sulfoxide. In addition, the present invention relates to a process for recycling racemic materials or those enriched in the undesired methanoanthrancenyl sulfoxide enantiomer which may be readily coupled with the enantioselective oxidation to produce pure enantiomeric material.

### SUMMARY AND DETAILED DESCRIPTION OF THE INVENTION

The present invention relates to novel optically active methanoanthrancenyl piperidinyl sulfoxide compounds and their pharmaceutically acceptable salts of the formula as described and set out on the first page following the Examples section wherein the various groups on the formula I (X, Y and R1) are defined as follows:
X and Y are independently selected from hydrogen, halogen and C1-6 alkoxy;
R1 is selected from:
Five- and six- membered heteroaryl rings containing from 1-3 heteroatoms selected from nitrogen, oxygen, and sulfur, and benz derivatives thereof, which may bear 1-2 substituents selected from C1-6 alkyl, hydroxy, C1-6 alkoxy which may bear a trifluoromethyl group, C1-6 alkoxycarbonyl, C1-6 hydroxyalkyl, benzyloxy, halo, C1-3 alkylaminocarbonylC1-3alkyl, aminocarbonyl having the formula: CONR^{c}R^{d} wherein R^{c} and R^{d} are independently selected from hydrogen, 2-pyrrolidinyl, and C₁₋₆ alkyl or wherein R^{c} and R^{d} together with the nitrogen atom to which each is attached form a 5- or 6- membered heterocyclic ring in which said nitrogen is the only heteroatom, R^{e}S(O)ₙ, R^{f}NH, and R^{g}S wherein R^{e} and R^{f} are independently selected from hydrogen and C₁₋₆ alkyl and n is 0, 1 or 2 and R^{g} is selected from C₁₋₃ alkylcarbonylaminophenyl and di(C₁₋₃)alkylarnino(C₁₋₆)alkyl wherein at least one of the substituents on the five- and/or six-membered heteroaryl ring is a chiral sulfoxide of the formula C₁₋₄alkylSO.

The invention further provides a pharmaceutical composition comprising a compound of formula I as defined above and shown on the pages following the examples and to pharmaceutically acceptable salts thereof and a pharmaceutically acceptable diluent or excipient. The claimed composition may be administered to a patient in need of treatment thereof in a suitable dosage form via an effective delivery system to provide D1 and D2 dopaminergic antagonist activity and to relieve or treat or prevent neurological and psychiatric disorders wherein antipsychotics are a prescribed treatment.

The invention further provides a method of treating neurological disorders comprising administering to a patient in need of treatment thereof a pharmaceutically effective amount of a compound according to formula I or a pharmaceutically acceptable salt thereof. The invention also relates to a method of treating psychosis comprising administering a pharmaceutically effective amount of a pharmaceutical composition containing an active ingredient as described in formula I and a pharmaceutically acceptable carrier or diluent. The invention further relates to a method of antagonizing a D1 and D2 dopamine receptor in humans comprising administering an antipsychotic amount of the compound according to formula I or a pharmaceutical composition containing the compound of formula I as the active ingredient.

In this specification, the terms "alkyl" and "alkoxy" include both straight and branched chain radicals, but it is understood that references to individual radicals such as "propyl" or "propoxy" embrace the straight chain radical and branched chains such as "isopropyl" or "isopropoxy" are referred to specifically.

The term "halo" is inclusive of fluoro, chloro, bromo, and iodo unless otherwise specified. The term "hydroxy" is given its ordinary meaning and may be included as a substituent as further shown herein. Particular values of C₁₋₆ alkyl include methyl, ethyl, propyl, isopropyl, butyl, isobutyl, sec-butyl, tert-butyl, pentyl, isopentyl, neopentyl, and isohexyl. Particular values of C₁₋₃ alkyl include methyl, ethyl, and propyl. Particular values of C₁₋₆ alkoxy include methoxy, ethoxy, propoxy, isopropoxy, butoxy, isobutoxy, sec-butoxy, tert-butoxy, pentoxy, isopentoxy, neopentoxy, hexoxy, and isohexoxy. Particular values of C₁₋₃ alkoxy include methoxy, ethoxy, and propoxy.

Particular values of five- and six-membered heteroaryl rings containing from 1-3 heteroatoms selected from nitrogen, oxygen, and sulfur include 2, 3- and 4-pyridyl, 2-pyrazinyl, 2- and 4-pyrimidinyl, 3- and 4-pyridazinyl, 3-, 4- and 5-isothiazoyl, 2-, 4- and 5-oxazolyl, 2-, 4- and 5-thiazolyl, 4- and 5-oxadiazoyl, 2- and 3-furyl, 2-, 4-and 5-imidazolyl, and 2- and 3-thienyl. The preceding rings can optionally bear the substituents listed above in the preceding paragraphs. Particular values of benz derivatives of five- and six-membered heteroaryl groups include the various quinolinyl, isoquinolyl, and benzothiazolyl groups which may also be substituted with halo or C₁₋₆ alkyl or alkoxy groups as shown above.

Particular values of C₁₋₆ hydroxyalkyl include hydroxymethyl, 1-hydroxyethyl, 2-hydroxyethyl, 1-3 hydroxypropyl, 1-hydroxyprop-2-yl, and 1-3 hydroxybutyl.

More particular values of X and Y include hydrogen and halo. More particular values of C₁₋₆ alkyl include C₁₋₄ alkyl, including methyl, ethyl, propyl, isopropyl, butyl, tert-butyl and isobutyl. More particular values of C₁₋₆ alkyloxy (alkoxy) include values of C₁₋₄ alkoxy, including methoxy, ethoxy, propoxy, isopropoxy, butoxy, isobutoxy and tert-butoxy. More particular values of five- and six-membered heteroaryl rings containing from 1-3 heteroatoms selected from nitrogen, oxygen and sulfur include 2, 3-, and 4-pyridyl, 3-, 4-and 5-isothiazoyl, 2-, 4- and 5-thiazoyl, and 2- and 4-imidazoyl wherein the rings may optionally be substituted with the substituents as defined above. More particular values of benz derivatives of five- and six-membered heteroaryl groups include 3-quinolyl, 4-isoquinolinyl, 2-methoxy-3-quinolyl, 2-benzothiazoyl, and 2-benzthienyl. More particular values of C₁₋₆ hydroxyalkyl include values of C₁₋₃ hydroxyalkyl such as hydroxymethyl, 1 or 2 hydroxyethyl, 1-3 hydroxypropyl.

The preferred values for X and Y as defined herein are hydrogen and chloro with hydrogen being most preferred. The preferred value of R¹ is as a six-membered heteroaryl ring substituted with 1-2 substituents and particularly includes 3-pyridyl substituted at the 2-position with C₁₋₄ alkylsulfinyl wherein the C₁₋₄ alkylsulfinyl lends chirality to the preferred compounds described herein.

A preferred compound may be selected from formula I as shown following the examples wherein:
X and Y are hydrogen;
R¹ is 3-pyridyl substituted at the 2-position with C₁₋₄ alkylsulfinyl. A specifically preferred compound is (-)-1-(9,10-Dihydro-9,10-methanoanthracen-9-ylmethyl)-4-(2-ethylsulfin yl-3-pyridyl)piperidin-4-ol. The present invention also includes enantiomeric mixtures of the above preferred compound wherein the (-) enantiomer is present in excess over the (+) enantiomer.

When X is chloro and Y is hydrogen, in general, 9S,10S stereochemistry may be preferred. The stereochemistry can be determined by standard methods such as coupling an acid chloride at the 9-position of the methanoanthracene ring with a chiral resolving reagent to produce a mixture of diastereomers which can be separated by recrystallization or via high performance liquid chromatography ("HPLC") or other chromatographic methods to produce the pure diastereomer whose absolute stereochemistry can then be determined. Diastereomeric adducts may also, in some circumstances, be separated by fractional crystallization.

The compounds produced and claimed herein may be selectively produced by chiral oxidation means of a sulfide moiety present on the described heteroaryl rings. The selective oxidation of the sulfide moiety to produce an enantiomerically enriched mixture wherein one enantiomer predominates as further described herein may also be coupled to a recycling process whereby the non-desired sulfinyl enantiomer may be reduced and reoxidized to form additional desired product. In general, a compound of formula I may readily be obtained by synthetic methods described herein. An alkylthio moiety may be prepared prior to the chiral oxidation and/or recycling procedure. A process for preparing a compound of formula I may proceed as follows:
(a) by treating a piperidone having formula II with a corresponding compound of formula R¹Li in an aprotic solvent such as THF. The lithium compound can be conveniently generated in situ by reacting a compound of formula R¹Z, wherein Z is a halo group or, in some cases hydrogen, with a C₁₋₆ alkyl lithium compound such as n-butyl lithium in a temperature range of -20 to -100 degrees C; the compound produced after coupling of the heteroaryl anion to the 4-piperidone methanoanthracenyl species is preferrably utilized as an intermediate (which is also part of the invention) which is further modified to form a compound of formula I wherein a C₁₋₄ alkylthio moiety is basified (to form an anion) and reacted with a leaving group on the five and/or six-membered heterocyclic ring wherein the resulting methanoanthrancenyl alkylthio intermediate (also part of the invention) is enantioselectively oxidized;
(b) by treating a corresponding amide of formula IIa with a suitable reducing agent, for example, lithium aluminum hydride or borane dimethylsulfide complex;
(c) by treating an aldehyde of formula III (G is hydrogen) with a corresponding piperidine of formula IV in the presence of a reducing agent such as, for example, sodium cyanoborohydride;
(d) for a compound of formula I wherein the value of C₁₋₆ alkoxy for either X or Y is desired, by treating a corresponding compound of formula I wherein X or Y is hydroxy with a desired base and alkylhalide to form the alkoxy compound(s).

The compounds produced in the above steps may be entioselectively oxidised using an enantioselective oxidising agent to produce the chiral sulphoxide of formula I.

In a further aspect of the invention there is provided a process for preparing a compound of formula I, or a pharmaceutically acceptable salt thereof, which comprises oxidising a compound of formula I, in which X and Y are as defined above and R¹ is as defined above with the exception that the or each of said groups of formula C₁₋₄alkylS0 is of formula C₁₋₄alkylS, via a chiral oxidation process so as to enantioselectively oxidize the said group of formula C₁₋₄alkylS to a chiral sulfoxide of the formula C₁₋₄alkylSO, thereby producing a compound of formula I; and where a pharmaceutically acceptable salt is required, treating the compound of formula I with an acid which forms a physiologically acceptable anion or a base which affords a physiologically acceptable cation.

In general it is preferred that the oxidation is carried out using a suitable oxidising agent which (as mentioned below) include, for example, a titanium/tartarate/peroxide mixture such as a mixture of a titanium alkoxide (eg. titanium tetraisopropoxide), a dialkyl tartarate (eg. diethyl tartarate) and an alkyl hydroperoxide (eg. t-butyl hydroperoxide). Other suitable oxidising agents include chiral oxaziridine such as (S)-(+)-camphorsulfonyl oxaziridine, and suitable enzymes. The reation is generally carried out in an inert hydrocarbon solvent such as toluene and is conveniantly carried out with cooling eg. in the range -78°C to ambient temperature.

Advantageously, the present invention relates to a process for producing a compound of formula I (as shown in the first page following the Examples) wherein X, Y and R¹ are defined as:
X and Y are independently selected from hydrogen, halogen and C1-6 alkoxy;
R1 is selected from:
Five- and six- membered heteroaryl rings containing from 1-3 heteroatoms selected from nitrogen, oxygen, and sulfur, and benz derivatives thereof, which may bear 1-2 substituents selected from C1-6 alkyl, hydroxy, C1-6 alkoxy which may bear a trifluoromethyl group, C1-6 alkoxycarbonyl, C1-6 hydroxyalkyl, benzyloxy, halo, C1-3 alkylaminocarbonylC1-3alkyl, aminocarbonyl having the formula: CONR^{c}R^{d} wherein R^{c} and R^{d} are independently selected from hydrogen, 2-pyrrolidinyl, and C₁₋₆ alkyl or wherein R^{c} and R^{d} together with the nitrogen atom to which each is attached form a 5- or 6- membered heterocyclic ring in which said nitrogen is the only heteroatom, R^{e}S(O)ₙ, R^{f}NH, and R^{g}S wherein R^{e} and R^{f} are independently selected from hydrogen and C₁₋₆ alkyl and n is 0, 1 or 2 and R^{g} is selected from C₁₋₃ alkylcarbonylaminophenyl and di(C₁₋₃)alkylamino(C₁₋₆)alkyl wherein at least one of the substituents on the five- and/or six-membered heteroaryl ring is a chiral sulfoxide of the formula C₁₋₄alkylS0; said process comprising the steps of:
   (a) forming a 9-formyl-9,10-dihydro-9,10 methanoanthracene of formula 22 by reacting a tricyclic aldehyde of formula 14 with a vinyl ester wherein the ester is vinyl-0(CO)(C₁₋₄)alkyl to form an ethanoanthracene acetoxy aldehyde of formula 16 or equivalent C₁₋₄ alkyl carbonyloxy aldehyde which is then treated with an oxidizing agent selected from CrO₃/H₂SO₄ in H₂O/acetone or an equivalent oxidizing reagent or system to form a corresponding 9-carboxylic acid shown as 18 or equivalent C₁₋₄alkyl carbonyloxy acid which is then treated with a diarylphosphoryl azide and a trialkylamine in a suitable solvent such as methylisobutylketone or equivalent to form a corresponding acyl (or C₁₋₄alkyl carbonyloxy) azide as a key intermediate which is then heated in MIBK or equivalent solvent to form a corresponding intermediate isocyanate which is then treated with excess aqueous sodium hydroxide (to reflux) or equivalent base and aqueous HC1 (heated) or equivalent acid to form an amino alcohol (as an HC1 or suitable salt) 20 which is then ring contracted via a carbocation intermediate (by a diazotization process or its equivalent) to form the formylmethanoanthracene 22; alternatively and less preferrably, the 9-carboxylic acid 18 or equivalent is treated with SOCl₂, DMF in toluent to form an intermediate acid halide which is then treated with NaN₃ (1.5 eq) to form the intermediate and corresponding azide which is then heated to form an isolable isocyanate which is then basified to form the amino alcohol 20 (isolated as the free amine from toluene or an equivalent solvent); alternatively, the interemediate isocyanate is acidified with an acetic acid/HC1 mixture in a solvent such as toluene or its equivalent to form the primary amine 20; the free amine produced in the alternative steps is then ring contracted via a carbocation intermediate to form 22;
   (b) forming a 4-piperidone of formula II by reacting the compound of formula 22 with a protected 4-piperidone wherein the protecting group is chosen from a ketal or other ketone protecting group known to those skilled in the chemical arts which is then reacted with a reducing agent to reduce the intermediate 9-ylmethanol to a 9-ylmethyl to form an intermediate and protected version of 22 which is then deprotected using a suitable acid to form II;
   (c) forming an intermediate compound of formula I wherein R¹ is equal to a five- and/or six-membered heteroaryl ring selected from:
Five- and six- membered heteroaryl rings containing from 1-3 heteroatoms selected from nitrogen, oxygen, and sulfur, and benz derivatives thereof, which may bear 1-2 substituents selected from C1-6 alkyl, hydroxy, C1-6 alkoxy which may bear a trifluoromethyl group, C1-6 alkoxycarbonyl, C1-6 hydroxyalkyl, benzyloxy, halo, C1-3 alkylaminocarbonylC1-3alkyl, aminocarbonyl having the formula: CONR^{c}R^{d} wherein R^{c} and R^{d} are independently selected from hydrogen, 2-pyrrolidinyl, and C₁₋₆ alkyl or wherein R^{c} and R^{d} together with the nitrogen atom to which each is attached form a 5- or 6- membered heterocyclic ring in which said nitrogen is the only heteroatom, R^{e}S(O)ₙ, R^{f}NH, and R^{g}S wherein R^{e} and R^{f} are independently selected from hydrogen and C₁₋₆ alkyl and n is 0, 1 or 2 and R^{g} is selected from C₁₋₃ alkylcarbonylaminophenyl and di(C₁₋₃)alkylamino(C₁₋₆)alkyl wherein at least one of the substituents on the five- and/or six-membered heteroaryl ring is a suitable leaving group which may be displaced by a C₁₋₄ alkylthio nucleophile;
   (d) forming an intermediate compound of formula I wherein the heteroaryl ring is as above and the C₁₋₄ alkylthio moiety has displaced the leaving group to form a compound of formula I wherein at least one of the substituents on the five- and/or six membered heteroaryl ring is a -S(C₁₋₄ alkyl) group;
   (e) reacting a compound produced in step (d) above with an enantioselective oxidizing agent under suitable conditions to form a compound of formula I.

The preferred compound produced in the above process and utilized in the pharmaceutical compositions of the invention is the crystalline form of (-)-1-(9,10-Dihydro-9,10 methanoanthracen-9-ylmethyl)-4-2-ethylsulfinyl-3-pyridyl)piperidin-4-ol. The preferred crystalline compound is characterized in that it has a melting point of 185-186 °C and has a specific rotation of [a] -106 ° (c = 1.0 in MeOH). The above preferred enantiomer is preferrably utilized as an antipsychotic. The use of the crystalline form of the compound (-)-1-(9,10-Dihydro-9,10 methanoanthracen-9-ylmethyl)-4-2-ethylsulfinyl-3-pyridyl)piperidin-4-ol for the treatment of psychoses in human patients is a feature of the claimed invention. The use of the crystalline form of the compound (-)-1-(9,10-Dihydro-9,10-methanoanthracen-9-ylmethyl)-4-2-ethylsulfinyl-3-pyridyl)piperidin-4-ol for the treatment of psychoses in human patients is a feature of the claimed invention characterized in that the crystalline compound is an antagonist of the D1/D2 dopamine receptors and the 5HT2 serotonin receptor. This balanced profile is predictive of an improved clinical profile since antipsychotic efficacy is achieved and side effects associated with D2 receptor antagonism are minimized.

A preferred process for producing crystalline (-)-1-(9,10-Dihydro-9,10 methanoanthracen-9-ylmethyl)-4-2-ethylsulfinyl-3-pyridyl)piperidin-4-ol is a feature of the claimed invention and comprises the steps of:
(a) forming a compound selected from 9-formyl-9,10-dihydro-9,10-methanoanthrancene by reacting a tricyclic aldehyde shown as 14 (X and Y = H) in Scheme 1 with vinyl acetate (>1 eq) in a sealed tube at 180°C to form an ethanoanthracene of formula 16 which is then treated with Jones reagent (CrO₃/H₂SO₄) in H₂O/acetone to form a corresponding 9-carboxylic acid 18 which is then treated with diphenylphosphoryl azide (>1 Eq) and triethylamine in methylisobutyl ketone at >80°C to form a corresponding acetoxyazide which is then heated in MIBK or equivalent solvent to form a corresponding intermediate isocyanate which is then treated with excess aqueous sodium hydroxide (to reflux) or equivalent base and aqueous HC1 (heated) or equivalent acid to form an amino alcohol shown as 20 (X and Y = H) and isolated as the HC1 salt which is then ring contracted via a carbocation intermediate (by diazotization using NaNO/₂HOAc in H₂O) to form 22 (X and Y = H); alternatively, the intermediately isocyanate is acidified with an acetic acid/HCl mixture in a solvent such as toluene to form the amino alcohol 20 which is ring contracted via the carbocation intermediate to form 22;
(b) forming a 4-piperidone of formula II (X and Y = H) by reacting a compound of formula 22 with a protected 4-piperidone wherein the protecting group is chosen from a ketal which is then reacted with a reducing agent selected from a boron reducing agent in a polar solvent to form a protected version of formula II which is then deprotected using a suitable acid to form II;
(c) forming an intermediate compound of formula I wherein R¹ is equal to 2-fluoro-3-pyridyl by reacting a compound of formula II with the anion generated by reacting 2-fluoropyridine with LDA;
(d) forming an intermediate compound of formula I wherein R¹ is equal to 2-ethylthio-3-pyridyl by reacting the thiolate anion generated by treating EtSH with sodium hydroxide or suitable base in a suitable solvent selected from 1-methylpyrollidone or equivalent with the compound generated in step (c) above; and
(e) treating the 1-(9,10-dihydro-9,10-methanoanthracen-9-ylmethyl)-4-(2-ethylthio-3-pyridyl)piperidin-4-ol generated in step (d) above with a cooled solution of diethyl D-tartrate in undried toluene or equivalent solvent; titantium tetraisopropoxide and t-butyl hydroperoxide to form an enantiomerically enriched ethylsulfinyl mixture which is then recrystallized in a suitable solvent such as MeCN or equivalent to form pure (-) enantiomer.

Thus, in a further aspect of the invention there is provided a process for producing crystalline (-)-1-(9,10-Dihydro-9,10-methanoanthracen-9-ylmethyl)-4-(2-ethylsulfinyl-3-pyridyl)piperidin-4-ol which comprises treating 1-(9,10-dihydro-9,10-methanoanthracen-9-ylmethyl)-4-(2-ethylthio-3-pyridyl)piperidin-4-ol with a solution (preferably cooled) of diethyl D-tartrate in toluene (preferably undried) or equivalent solvent; titantium tetraisopropoxide and t-butyl hydroperoxide to form an enantiomerically enriched ethylsulfinyl mixture which is then preferably recrystallized in a suitable solvent such as MeCN or equivalent to form pure (-) enantiomer.

If not commercially available, the necessary starting materials for the procedures such as those described above may be made via standard synthetic organic chemical technology or from known methods used to produce analogous compounds in the synthetic chemical sequence. For example, compounds with the formula R¹Z can be made by techniques similar to that described in Brandsma et al., Preparative Polar Organometallic Chemistry I, Springer-Verlag (1987). In the discussion which follows various standard chemical abbreviations and acronyms have been employed. For example, Et is ethyl, THF is tetrahydrofuran, ^{t}Bu is tert-butyl, RT is room temperature, DMSO is dimethylsulfoxide, Me is methyl, Cbz is carbobenzyloxy and Ph is phenyl. Z is a halo group such as chloro when Grignard agents or alkyllithium compounds are described.

A common intermediate for making compounds according to the invention is an acid (G is hydroxyl) or acid halide ( G is a halo group such as chloro) of formula III. This intermediate can be made as shown in Scheme 1 which is illustrated following the examples. An anthraquinone of formula 10 can be reduced to the corresponding anthracene of formula 12 using zinc and aqueous ammonia. Anthracene 12 can then be converted to the corresponding 9-aldehyde 14 using phosphorous oxytrichloride and N-methyl formanilide. Reaction of aldehyde 14 with vinyl acetate(Diels Alder reaction) affords the bridged compound 16 which can then be oxidized with chrominum trioxide (in the presence of sulfuric acid) to the corresponding acid 18. Acid 18 can then be successfully treated with thionyl chloride (in, for example, toluene) to make the corresponding 9-acid chloride, followed by sodium azide (in, for example, a mixture of water and acetone) to make the corresponding 9-acyl azide, followed by heating (in, for example, toluene) to effect rearrangement to the corresponding isocyanate, followed by treatment with an alkali metal hydroxide (in an alcohol such as ethanol) to cleave the acetyl group to hydroxy and hydrolyze the isocyanate to amino, thereby yielding the amine 20. Amine 20 can then be treated with an alkali metal nitrite (for example, sodium nitrite in acetic acid) to effect a ring contraction and thereby yield the 9-aldehyde of formula 22. Aldehyde 22 can then be oxidized with, for example, chromium trioxide in the presence of sulfuric acid, to yield the corresponding acid of formula 24 (corresponding to the acid of formula III wherein G is hydroxyl). The corresponding 9-acid halide such as the acid chloride may then be obtained by treating acid 24 with thionyl halide (thionyl chloride) or oxalyl halide (chloride).

If a 2,7-dihalo substituted methanoanthracene is desired it may be prepared according to the following general procedure. An unresolved acid 24 ( or resolved if an optically enriched disubstituted product is desired) which is mono-substituted at the 2-position with a desired halo substituent is reacted with a thionyl halide to make the corresponding 9-acid chloride. This may then be reacted with a lower alcohol such as methanol or ethanol to afford a 9-alkyl ester. The 2-halo ester can then be nitrated at the 7-position by reaction with a suitable nitrating agent such as a combination of trifluoroacetic anhydride and ammonium nitrate under an inert gas (e.g. nitrogen) atmosphere. This reaction will generally produce a mixture of 2-halo 6 or 7 nitro positional isomers which can be separated by conventional separation techniques. The 2-halo-7-nitro isomer can be reduced to the corresponding 7-amino-2-halo compound by a suitable reducing agent such as stannous chloride which can be further converted to the corresponding 2,7-dihalo alkyl ester by reaction with a diazotizing agent such as tert-butyl nitrite followed by treatment with cupric halide. The ester can then be cleaved with a suitable base (such as alkali metal hydroxide) to afford the corresponding 2,7-dihalosubstituted acid.

If an oxygenated substituted methanoanthracene is desired (e.g. 2-halo-7 alkoxy) it can be prepared staring with the 7-amino-2-halo compound(s) described above. The amine is treated with a diazotizing agent such as tert-butyl nitrite followed by treatment with the salt of a suitable acid such as trifluoroacetic acid ( the salt being formed, for example, with potassium carbonate in trifluoroacetic acid as the solvent). The resulting trifluoroacetate can then be hydrolyzed by conventional means and C₁₋₆ alkyl groups attached to the oxygen by treatment with base in the presence of a corresponding C₁₋₆ alkyl halide.

An amide of formula IIa can be made by treating an acid chloride of formula III (G is halo) with a piperidine of formula IV in the presence of a base such as a trialkylamine, for example triethylamine. A piperidone of formula II can be made, as illustrated in Scheme 2 by oxidizing a corresponding hydroxypiperidine of formula 32 using an appropriate oxidizing agent such as (1) chromium trioxide in the presence of sulfuric acid and using a suitable solvent such as acetone; (2) sulfur trioxide-pyridine complex in the presence of a base such as trialkylamine (e.g. triethylamine) and using a suitable solvent such as a combination of methylene chloride and DMSO; or (3) a combination of oxalyl chloride and DMSO followed by treatment with a base such as a trialkylamine and using a solvent such as methylene chloride.

A hydroxypiperidine of formula 32 can be made by either of the routes illustrated in Scheme 2. 9-aldehyde 22 can be treated directly with 4-hydroxypiperidine and sodium cyanoborohydride to yield the corresponding amine 32. Alternatively, 9-aldehyde 22 can first be oxidized and converted to the corresponding 9-acid chloride as previously described, followed by treatment with 4-hydroxypiperidine, wither in excess or with added base such as a trialkylamine (for example, triethylamine) to yield the corresponding amide 30. Reduction of amide 30 with LAH in diethylether or THF can then be conducted to afford hydroxypiperidine 32.

Piperidines having formula IV can be synthesized as illustrated in Scheme 3. 4-hydroxypiperidine 50 can be reacted with carbobenzyloxy chloride (Cbz-Cl) or other suitable protecting group in the presence of a base such as Et₃N to protect the piperidino nitrogen and thereby yield the corresponding 1-(carbobenzyloxy)piperidine-4-ol 52. Oxidation of piperidine-4-ol 52 with oxalyl chloride and DMSO followed by treatment with a base and in a solvent such as methylene chloride yields the corresponding protected 4-piperidone 54. Piperidone 54 can be treated with an organolithium compound R¹Li wherein R¹ is as defined previously or a Grignard reagent R¹MgZ, at a temperature of -20 to -70 degrees C and in a solvent such as THF or diethylether to yield the corresponding protected hydroxypiperidine 56. 56 can readily be deprotected by treatment with a palladium-on-carbon catalyst (for example 10% Pd/C) and cyclohexene in a solvent such as ethanol, thereby yielding the desired hydroxypiperidine of formula IV. It is noted that many of the materials for synthetic methods as described above and herein are commercially available and/or widely reported in the synthetic literature

The C₁₋₄ alkylthio derivatives used as precursors to the preferred chiral sulfoxides of the instant invention may readily be prepared according to the following basic procedure. To a solution of, for example, 1-(9,10-dihydro-9,10-methanoanthracen-9-ylmethyl)-4-(2-fluoro-3-pyridyl)piperidin-4-ol ( which is prepared from reacting the anion of 2-fluoropyridine with 1-(9, 10-dihydro-9, 10-methano-anthracen-9-ylmethyl)-4-piperidone and lithium bromide) in a suitable solvent such as THF is added the sodium salt of a C₁₋₄ alkylthio compound which displaces the flouro group. The sodium alkylthiolate is readily prepared from the C₁₋₄ alkylthiol compound and sodium hydride under standard conditions. The reaction between the alkylthiolate and the 2-fluoro derivative is allowed to proceed under reflux for about a minimum of three hours and is subsequently quenched by pouring into water. The aqueous phase is extracted with ether or other suitable organic solvent and the resultant product is purified by suitable chromatographic means such as flash chromatography to obtain a 1-(9,10-dihydro-9,10-methanoanthrancen-9-ylmethyl)-4-(2-C₁₋₄ alkylthio-3-pyridyl)piperidin-4-ol compound. The preferred compound produced in this manner is the 2-ethylthio derivative. Of course, a suitable flouro-substituted heteroaryl compound as described herein may also be reacted with an alkylthiolate salt to produce suitable precursors to the claimed chiral sulfoxides.

The above 2-ethylthio derivative or other C₁₋₄ alkylthio derivative as described herein may then be oxidized enantioselectively to form a desired and biologically active dopamine receptor antagonist. The selective oxidation procedure of the described alkylthio compounds may proceed as follows. To a cooled solution (-20 degrees C) of (-)-diethyl D-tartrate in a suitable solvent such as dry (anhydrous) toluene under a nitrogen atmosphere is added titanium tetraisopropoxide in a dropwise fashion. The mixture is stirred for about five minutes with the subsequent addition of an alkylthio derivative such as 1-(9, 10-dihydro-9,10-methanoanthracen-9-ylmethyl)-4-(2-ethylthio-3-pyridyl)piperidin-4-ol which is added in one portion. The reaction is maintained at the above temperature for about 25 minutes and is then cooled to about -78 degrees C before the addition of freshly dried t-butyl hydroperoxide (in slight molar excess) via syringe. The reaction is allowed to warm to -15 degrees C over a several hour period (about 3 hours) and is then placed without stirring in a 15 degree freezer for about 18 hours. The reaction is then quenched by adding aqueous sodium hydroxide (about 1N). The suspension is then diluted with methylene chloride and the mixture is filtered through diatomaceous earth. After work-up, the product is purified by chromatographic means such as flash chromatography to give an enantiomeric mixture of chiral sulfoxides wherein the enantiomeric ratio is about 1:3.7 of (+) C₁₋₄ alkylsulfinyl methanoanthrancenyl compound to (-) C₁₋₄ alkylsulfinyl methanoanthracenyl compound ( as determined by HPLC analysis of the enantiomers). Recrystallization of the product mixture in a suitable solvent such as hot toluene leads to an enantiomeric mixture with a ratio of 1:99 wherein the (-)-enantiomer predominates in the mother liquors. Crystallization of the mother liquor leads to a pure (-)-enantiomer as a crystalline solid. Scheme 4 depicts the specific chiral oxidation(s) of either the pyridyl ethyl thio compound or the heteroaryl (het) C₁₋₄ alkyl thio compound(s) to form the respective sulfinyl compound. In Scheme 4, het refers to the five and six membered heteroaryl rings as defined herein for R1. The preferred enantiomer produced in this manner is (-)-1-(9, 10-Dihydro-9, 10-methanoanthrancen-9-ylmethyl) -4-(2-ethylsulfinyl-3-pyridyl)piperidin-4-ol. The present invention therefore relates to a process for producing chiral sulfinyl methanoanthracenyl compounds comprising the steps of (a) reacting a compound of the formula I wherein X and Y and R¹ are as defined herein except R¹ includes a C₁₋₄ alkylthio moiety with (b) an asymmetric oxidizing reagent selected from (1) titanium/tartrate/peroxide or (2) a chiral oxaziridine wherein the reaction proceeds enantioselectively to produce a chiral enantiomeric mixture of oxides of formula I. The present invention also relates to a process comprising the steps as described above plus an additional recrystallization to afford an enantiomerically pure sulfinyl enantiomer of formula I. Of course, the invention also encompasses those diastereomeric mixtures which result wherein at least one of the heteroaryl substituents is a chiral sulfinyl moiety and an additional chiral substituent is present on the heteroaryl group as defined herein or on the methanoanthracenyl nucleus. The present process produces significant enantiomeric excess and thus the present invention is directed to a novel process for producing enantiomeric methanoanthracenyl sulfoxides. A process for asymmetrically oxidizing aryl-alkyl sulfides of specific formulae to sulfoxides is described generally in Pitchen et al. J. Am. Chem. Soc. 1984, 106, 8188-8193. An oxaziridine procedure is generally described in Davis et al., J. Am. Chem. Soc. 1982, 104 5412. The processes described herein are directed to chiral sulfide oxidations of complex methanoanthracenyl alkyl sulfides. Other chiral oxidation processes which could be utilized under appropriate conditions include enzymatic means (via chloroperoxidase) or oxidation in the presence of bovine serum albumin. Enantiomeric ratios could vary also depending upon the particular heteroaryl moiety.

The present invention also relates to a process whereby the non-desired sulfoxide is reduced back to the sulfide which is then prepared for an additional enantioselective oxidation to produce the preferred enantiomer or diastereomer. For example, a compound such as a)1-(9,10-dihydro-9,10-methanoanthracen-9-ylmethyl)-4-(C₁₋₄alkylthio-3 -heteroaryl)piperidin-4-ol derivative may be synthesized by reducing the corresponding C₁₋₄alkylsulfinyl-3-heteroaryl derivative with a suitable reducing agent such as zinc dust/titanium tetrachloride. The preferred compound recycled in this manner is the racemate of or material containing the (+)-1-(9,10-dihydro-9,10-methanoanthrancen-9-ylmethyl)-4-(2-ethylsulfinyl-3-pyridyl)piperidin-4-ol which, after reduction, yields 1-(9,10)-dihydro-9,10-methanoanthrancen-9-ylmethyl)4-(2-ethylthio-3-pyridyl)piperidin-4-ol. Advantageously, this compound is then oxidized via the enantioselective process described herein to form additional (-)-enantiomer. Therefore, the present invention relates to a selective and dual process for preparing specific enantiomers or diastereomers of C₁₋₄ alkylsulfinyl methanoanthrancenyl compounds via a selective oxidation and recycling procedure. This process yields specific enantiomers or diastereomers which are useful as D1/D2 dopamine receptor antagonists such as the preferred (-)-1-(9,10-dihydro-9,10-methanoanthracen-9-ylmethyl)-4 -(2-ethylsulfinyl-3-pyridyl)piperidin-4-ol compound. This compound has a surprisingly improved D1/D2 ratio which can generally be indicative of an improved clinical therapeutic profile in patients in need of treatment of disorder such as psychoses. Its D1 receptor antagonist activity is closer to its D2 receptor antagonist activity thus leading to a balanced D1/D2 profile which may be predictive of an improved clinical profile over conventional antipsychotic agents. Surprisingly, the (-)-enantiomer has an improved D1/D2 profile over the racemate of the same chemical formula.

Examples of suitable pharmaceutically acceptable salts are organic addition salts formed with acids which form a physiologically acceptable anion, for example, tosylate, methansulfonate, acetate, tartrate, citrate, succinate, benzoate, ascorbate, fumurate, maleate, alpha-ketoglutarate, and alpha-glycerophosphate. Suitable inorganic salts may also be formed such as sulfate, nitrate, phosphate and hydrochloride. Pharmaceutically acceptable salts may be obtained using standard procedures well known in the art, for example, by reacting a compound of formula I with a suitable acid to afford a pharmaceutically acceptable salt.

When used to treat psychoses or other neurological disorders as recited herein, a compound of formula I or preferably the compound (-)-1-(9,10)-Dihydro-9,10-methanoanthracen-9-ylmethyl)-4-(2-ethylsulfi nyl-3-pyridyl)piperidin-4-ol is preferably used. This compound may be admixed with a pharmaceutically acceptable carrier or diluent and administered in a pharmaceutically effective amount to a patient in need of treatment thereof. The particular composition chosen may be dependent upon or adapted for the desired route of administration. Of course, these suitable pharmaceutical compositions are a further feature of the invention described herein. These compositions may be obtained by utilizing conventional procedures and by incorporating conventional pharmaceutical binders, excipients, fillers, inert ingredients, disintegrants and the like. The active compounds and/or the pharmaceutical compositions described herein may be prepared in a variety of dosage forms depending upon the preferred route of administration. Oral dosage forms may include tablets, capsules, solutions or suspensions. Intravenous, intravesicular, subcutaneous, or intramuscular injection or infusion dosage forms may be in the form of sterile solutions or suspensions. Suppository compositions may be prepared if rectal administration is necessary. Oral administration is the preferred route of administration for soluble salts within the scope of the present invention. Advantageously, the less soluble free amines or non-salt forms within the scope of the present invention are administered to a patient in a sustained release or DEPOT formulation wherein the active ingredient is slowly released over a period of days or other suitable period to a patient in need of treatment thereof. The present invention therefore also relates to a method of administering a therapeutically effective amount of the active compounds as disclosed herein via oral or DEPOT delivery means. Sustained release formulations and drug delivery means are generally described in Remingtons Pharmaceutical Sciences. There is a need for a long acting dosage form in the treatment of psychoses with sustained release properties capable of providing therapeutic 5HT2 serotonin, D1 and D2 dopamine antagonist plasma concentrations.

A DEPOT formulation containing the described active ingredients may be administered by implantation (for example, subcutaneously, transcutaneously or intramuscularly) or by intramuscular injection. These formulations may therefore be formulated with pharmaceutical excipients such as suitable polymeric or hydrophobic materials (e.g. as emulsions in oil) or as a sparingly soluble salt. The dosages of the DEPOT formulation are chosen to administer a pharmaceutically effective amount of the described D1/D2/5HT2 antagonist over an extended period of time.

The dose of a compound of formula I which is administered to a patient in need of treatment thereof will necessarily vary depending upon the particular patient and the severity of their condition and their body weight and particular biochemistry. A clinician or physician of ordinary skill in the art can readily determine the suitable dosage from the ranges described herein and from the patients particular characteristics. In general, a compound of formula I will be administered to a patient in need of treatment thereof (such as man) so that a pharmaceutically effective amount is received and this dosage is generally in the range of about 0.01 to about 10 mg/kg body weight. If a drug is administered intramuscularly, it is administered in the range of about 0.01 to about 5 mg/kg body weight. If it is administered orally, it is administered in the range of about 0.1 to about 10 mg/kg body weight. It will be apparent to those skilled in the art that a compound of formula I can be co-administered with other therapeutic or prophylactic agents and/or medicaments that are not contraindicated therewith.

The compounds of formula I are antagonists of both D1 and D2 dopamine receptors and therefore are useful as antipsychotic drugs. In addition, the compounds of formula I are useful as 5HT2 serotonin receptor antagonists and thus are further useful as antipsychotics. These compounds therefore have significant serotonin antagonist activity as well as a balanced D1/D2 profile. D1 and D2 and 5HT2 antagonism may be shown by standard tests as described below. D2 antagonism can be shown by standard tests such as antagonism of [³H]-spiperone binding and/or antagonism of apomorphine-induced climbing and apomorphine-induced disruption of swimming. Additional tests are described below. The following are non-limiting examples of the antagonism of the compounds of the invention on the D1, D2 and 5HT2 receptors and clearly show an improved and balanced receptor antagonist profile.

### TEST A

Receptor binding assays used to quantitate the affinities of compounds for the Dopamine D-1, Dopamine D-2 and Serotonin 5HT2 receptors were performed according to Saller and Salama (J Pharm Exp Ther 236 pg. 714, 1986) and Saller et al. (J Pharm Exp Ther 253 pg. 1162, 1990) with minor modifications. In brief, the Dopamine D-1 binding assays were carried out at 37°C for 15 minutes in 1.0 ml of 50 mM Tris-HCl containing 120 mM NaCl, 5 mM KCl, 2mM CaCl2 and 1 mM MgCl2 (pH=7.4), membranes from 3 mgs tissue from rat striatum and 0.3 nM [³H]SCH 23390. The Dopamine D-2 binding assays were carried out at 37°C for 15 minutes in 1.0 ml of 50 mM Tris-HCl (pH=7.7) containing 40 nM ketanserin, membranes from 4 mgs tissue from rat striatum and 0.1 nM [³H]Spiperone. The Serotonin 5HT2 binding assays were carried out at 37°C for 15 minutes in 1.0 ml of 50 mM Tris-HCl (pH=7.7), membranes from 5 mgs tissue from rat frontal cortex and 0.5 nM [³H]Ketanserin. All assays reactions were terminated by rapid filtration over Whatman GF/B filters. Radioactivity on the filter was determined by scintillation counting. Nonspecific binding for D-1, D-2 and 5HT2 assays was defined as the binding activity in the presence of 1 µM SCH 23390, butaclamol or ketanserin, respectively, and was subtracted from the total binding to determine specific binding. Each assay was carried out at least three times in triplicate. IC50 values were determined from a least squares regression of a logit-log transformation of the data. Ki values were calculated from the determinations using the following equation: Ki=IC50 / (L+Kd), where L=ligand concentrations used in the assays and Kd=equilibrium dissociation constant of the radioligands as determined from saturation experiments. The Kᵢ values of, for example, racemic 1-(9,10-dihydro-9,10-methanoanthracen-9-ylmethyl)-4-(2-ethylsulfinyl-3 -pyridyl)piperidin-4-ol were 118 nM, 10 nM, and 21 nM for the D1, D2 and 5HT2 receptors respectively. The pure (about 100%) levorotatory isomer of the above compound gave 13 nM, 42 nM and 39 nM.

### TEST B

Female Swiss-Webster mice weighing approximately 20 g were deprived of food for approximately 24 h and then dosed orally with various doses of either the vehicle or test agent over a range of doses (N=20 mice per treatment group). Thirty minutes later they were dosed with amorphine HCl at 1.25 mg/kg, sc, and placed into climbing cages. The climbing cages were 9 cm side, 15 cm deep and 30 cm high. One wall had 27 horizontal rungs spaced 1 cm apart. Thirteen minutes after apomorphine administration, each mouse was observed continuously for 1 min and the highest and lowest rung reached by its front paws was recorded. The mean of these two scores was used as the score for that mouse (the highest and lowest scores were 27 and 0, respectively). Immediately after the 1-min climbing observation period each mouse was placed into a circular swimming tank for 2 min and the number of swims was counted. The height of the tank was 15 cm and the diameter was 28 cm. A circular obstacle measuring 10.5 cm in diameter and having a height of 17 cm was placed into the center of the tank, creating a circular swimming channel 8.75 cm wide. The water level was 5.5 cm and the water was kept at room temperature. Marks were placed on the floor and the side of the tank 180 degrees apart. A "swim" was scored each time a mouse swam from one mark to the other. The mice were observed through overhead mirrors and the number of 180 degree swims was recorded for each mouse. Activity in this test was indicated by a decrease in the climbing score accompanied by an increase in the swimming score at a given dose of the test compound. Results are expressed as ED50s for climbing and swimming,.i.e., dose at which there was a 50% decrease in climbing and dose at which there was a 50% increase in swimming. Typical results for climbing and swimming respectively were ED50s of 0.54 and 1.7 mg/kg po for the racemate described above and 1.3 and 1.6 mg/kg po for the pure levorotatory isomer.

### TEST C

Ten µg of 6-hydroxydopamine dissolved in 2 µl physiological saline containing 0.1 mg/ml ascorbic acid was injected into the left substantia nigra of male Sprague Dawley rats under sodium pentobarbital anesthesia at least one month prior to the experiment, according to the method of Perese et al. (Brain Research, 494, pg 285, 1989). The stereotaxic coordinates from Bregma were: AP -5.2, L +2.0, V -7.5. Ipsiversive and contraversive rotational behavior was monitored using Plexiglas cylinder rotometers (Columbus Instruments; Rota-Count-8) and the number of rotations made in 5 minute intervals was recorded for the entire duration of drug effect. Animals exhibiting >500 contralateral rotations / 2.5 hour post-injection period in response to L-DOPA (50 mg/kg i.p.; t=0) and benzerazide (30 mg/kg i.p.; t=-15 min) were used for further testing. Contraversive rotational behavior also was dose-dependently induced by the Dopamine D-1 selective agonist, SKF 38393. We examined the antagonism of a subthreshold dose (20 mg/kg i.p.) of SKF 38393-induced circling behavior with the racemate described above and the respective 95% pure isomer. SKF 38393 and the racemate or the 95% pure isomer were administered simultaneously (t=0) and rotational behavior was measured for 60 minutes. We found that the racemate described above had an IC50 value (drug concentration which produced a 50% inhibition of SKF 38393-induced rotations) of 3.95 mg/kg (i.p.) and the member of the heteroaryl C₁₋₄ alkyl sulfinyl class described above ( 95% pure C₁₋₄ alkyl sulfinyl levorotatory isomer) had an IC50 value of 1.98 mg/kg (i.p.).

### Test D

Male Sprague Dawley rats weighing 60-120 g were deprived of food for approximately 24 h and then dosed orally with either vehicle or test agent over a range of doses (N=9 rats per treatment group). Twenty minutes later they are dosed with quipazine dimaleate, 2.5 mg/kg i.p. and placed singly in plexiglass chambers. Five minutes after dosing with quipazine, the number of head twitches are counted for a fifteen minute period. Results are analyzed by determining mean head twitch scores and percent inhibition for the drug treated vs the vehicle treated groups and reported as ED50s, i.e., dose at which there is a 50% reduction in head twitch scores. Typical results in this test were ED50s of 0.61 mg/kg po for the racemic agent and 1.38 mg/kg po for the pure isomer (100%).

The invention will now be illustrated by the following non-limiting synthetic examples in which, unless stated otherwise:
(i) temperatures are given in degrees Celsius (C); operations and/or reactions were carried out at room or ambient temperature, that is, in the range of 18-25 °C.
(ii) evaporation of solvent was carried out using a rotary evaporator under reduced pressure (600-4000 pascals; 4.5-30 mmHg) with a bath temperature of up to 60 °C;
(iii) flash chromatography was carried out on Merck Kieselgel (Art 9385) or Baker Flash Silica Gel; thin layer chromatography (TLC) was carried out on Analtech 0.25 mm silica gel GHLF plates (Art 21521), obtainable from Analtech, Newark, DE, USA;
(iv) High pressure liquid chromatography (HPLC) for analysis of enantiomeric purity determinations of the chiral species described herein was carried out on either a 25cm x 4.6mm Chiralcel OD or a 15cm x 4.6 mm Ultron Ovomucoid column available from JT Baker, Inc.; HPLC analyses for most reactions and final products was carried out on either a 25cm x 4.6 mm Supelcosil LC-8-DB, a 25 cm x 4.6 mm Supelcosil LC-18-DB column available from Supelco, State College, PA, USA or a 25 cm x 4.6 mm Zorbax RX column.
(v) in general, the course of reactions was followed by TLC and/or HPLC and reaction times are given for illustration only;
(vi) melting points are uncorrected and (dec) indicates decomposition; the melting points given are those obtained for the materials prepared as described; polymorphism may result in isolation of materials with different melting points in some preparations;
(vii) all final products were essentially pure by TLC and/or HPLC and had satisfactory nuclear magnetic resonance (NMR) spectra and microanalytical data;
(viii) yields are given for illustration only;
(ix) reduced pressures are given as absolute pressures in pascals (Pa); other pressures are given as gauge pressures in bars;
(x) chemical symbols have their usual meanings; the following abbreviations have also been used: v (volume), w (weight), mp (melting point), L (liters), mL (milliliters), g (grams), mmol (millimoles), mg (milligrams), min (minutes), h (hour); and
(xi) solvent ratios are given in volume; volume (v/v) terms.

### EXAMPLES

### Example 1-Method A

### (-)-1-(9,10-Dihydro-9,10-methanoanthracen-9-ylmethyl)-4-(2-ethylsulfin yl-3-pyridyl)piperidin-4-ol

### (titanium/tartrate/hydroperoxide based oxidation)

To a cooled solution (-20 °C) of (-)-diethyl D-tartrate (18.55 g. 90.5 mmol) in dry toluene (425 mL) under a nitrogen atmosphere was added titanium tetraisopropoxide (14.47 mL, 45.25 mmol) in a dropwise fashion. After stirring for five minutes, solid 1-(9,10-dihydro-9, 10-methanoanthracen-9-ylmethyl)-4-(2-ethylthio-3-pyridyl)piperidin-4-o 1 (20.0 g, 45.25 mmol) was added in one portion. The solution was maintained at -20 °C for 25 minutes and cooled to -78 °C (dry ice/acetone) prior to the addition of freshly dried t-butyl hydroperoxide (90%, 5.28 mL, 47.5 mmol) in a dropwise fashion via syringe. The flask was allowed to warm to -15 °C over three hours, stirring was discontinued and the reaction vessel was placed in a -15 °C freezer for 18 hours. The reaction was quenched by the addition of aqueous sodium hydroxide (1.0 N, 400 mL). The suspension was diluted with methylene chloride (500 mL) and the resulting mixture was filtered through diatomaceous earth, rinsing with additional methylene chloride. The organic layer was separated. The aqueous phase was extracted with methylene chloride (2 X 200 mL). Combined organic extracts were dried over anhydrous sodium sulfate, filtered and reduced.

The product was preabsorbed onto silica gel in preparation for purification by flash chromatography over silica gel (1000 mL, eluent: 20% methanol/ethyl acetate ramped to 30% as the desired compound elutes) to yield 17.63 g (85 %) of the title compound as a yellow/white solid. TLC analysis (sulfoxide: Rf 0.24, sulfone/sulfide Rf 0.73, eluent: 15% methanol/ethyl acetate). The enantiomeric purity of this compound was determined to be 1:3.7 ((+):(-)) by HPLC analysis (Ultron ES-OVM 6.0 x 15 cm, mobile phase 80% 0.01 M pH 7.0 phosphate buffer/20% acetonitrile, flow rate: 1.0 mL/min, maximum 10 uL injection at 2.0 mg/ml; retention time: isomer 1 13.4 min, isomer 2 15.7 min). The solid material was dissolved in hot toluene and cooled to room temperature and seeded with several crystals of racemic sulfoxide. The racemate crystallizes slowly over 72 hours. The solid was removed by filtration, recovering 11.73 g of sulfoxide as an oil at the 5:95 purity level (isomer (+)/(-) ratio as determined by HPLC). This oil was dissolved in hot toluene and allowed to crystallize for an additional 24 hours. The oil (7.7 g) was dissolved in toluene at room temperature, filtered and seeded with crystals of pure isomer (-). After 4 hours at room temperature, the crystallization vessel was placed in the freezer at -15 °C overnight. The resulting solid slurry is diluted with cold toluene (0 °C, 15 mL) and filtered. The white solid was dried at high vacuum to remove remaining solvent. This procedure formed pure isomer (-) (6.9 g, 33.3 % yield of a high purity state by HPLC analysis, mp 185-186 C (racemate: 206-208 C), specific rotation [a] -106 (c=1.0, MeOH) degrees per gram per mL per dm ((+)-isomer specific rotation average +117, c=1.0, MeOH).

The free base was dissolved in methylene chloride, acidified with ethereal HCl and diluted with ether. The resulting hydrochloride salts were filtered, rinsed with fresh ether, and dried in vacuo (55 °C, 10 pascal, 18 h) to yield off-white solids, mp sulfoxide 213-216-°C (dec). hydrochloride salt: 1H NMR (d6-DMSO, 300 MHz) 10.19 (br s, 1H), 8.69 (d, J=4.4 Hz, 1H), 7.63 (br d, J=7.9 Hz, 1H), 7.57 (m, 1H), 7.37 (m, 4H), 7.00 (m, 4H), 4.44 (m, 3H), 3.58 (m, 4H), 3.11 (m, 1H), 2.86 (m, 1H), 2.76 (br s, 2H), 2.30 (br m, 2H), 1.97 (br m, 2H), 1.18 (t, J=7.5 Hz, 3H) MS (CI, CH4) m/z 459 (M+1,100), 487 (M+29,16), 441 (21), 413 (12)

| Analysis for C₂₈H₃₀N₂O₂S.1.8 HCl | | | |
|---|---|---|---|
| Calculated | C, 64.15; | H, 6.11; | N, 5.34 |
| Found | C, 64.27; | H, 6.46; | N, 5.23 |

### Example 1-Method B (oxaziridine based oxidation)

To a cooled solution (-78 °C) of 1-(9,10-dihydro-9,10-methanoanthracen-9-ylmethyl)-4-(2-ethylthio-3-pyridyl)piperidin-4-ol (200 mg, 0.452 mmol) in dry chloroform (or toluene) (8 mL) under a nitrogen atmosphere was added (S)-(+)-camphorsulfonyl oxaziridine (104 mg, 0.452 mmol). The cooling bath was removed and the reaction slowly warmed to room temperature. Conversion was monitored by thin layer chromatography (TLC analysis: sulfoxide Rf 0.24, sulfone/sulfide Rf 0.73, eluent: 15% methanol/ethyl acetate). After several hours starting material was still evident and could not be oxidized by the addition of more oxidant. The reaction was quenched by the addition of aqueous sodium hydroxide (1.0 N, 10 mL). The aqueous phase was extracted with methylene chloride (2 X 15 mL. Combined organic extracts were washed with water, dried over anhydrous sodium sulfate, filtered and reduced.

The product was purified by flash chromatography over silica gel (50 mL, eluent: 10% methanol/ethyl acetate) to yield 93 mg (45 %) of the title compound as a yellow/white solid. The enantiomeric purity of this compound was determined to be 1:5.6 ((+):(-)) by HPLC analysis. The (-)-isomer could be further enriched by the procedure described above.

The starting piperidin-4-ol was prepared as follows:
a. 9,10-Dihydro-9,10-methano-9-anthracenecarboxylic acid
To a cooled solution (0 °C) of 9-formyl-9,10-dihydro-9, 10-methanoanthracene (literature preparation: M. Sunagawa, et al.; Chem. Pharm. Bull. Vol. 27 (1979) pp 1806-1812; U.S. Patent 4,224,344 Sunagawa et al., Sumitomo, Ltd.; Sept. 23, 1980; U.S. Patent 4,358,620 Sunagawa et al., Sumitomo, Ltd.; Nov. 9, 1982) (78.5 mmol) in acetone (260 mL) was added Jones reagent (24 mL; 27 g chromium trioxide, 23 mL water diluted up to 100 mL of reagent solution) in portions. The reagent was added until an orange color persists. The reaction, containing a significant amount of reduced chromium salts, was warmed to room temperature. The solvents were removed in vacuo and replaced with water (300 mL) and saturated with sodium chloride. The aqueous phase was extracted with ethyl acetate (3 x 300 mL). Combined organic extracts were extracted with 2.5 N NaOH (3 x 400 mL). The basic aqueous extracts were acidified with 3 N HCl, saturated with sodium chloride, and extracted with ethyl acetate (4 x 300 mL). Combined organic extracts were dried over anhydrous magnesium sulfate, filtered, and reduced to a white solid. The title compound was formed in 80% yield as a white solid. MS (Cl CH4) m/z 237 (M+1,100), 265 (m+29,10), 219 (22), 209 (15), 193 (20)
b. 1-(9,10-Dihydro-9,10-methanoanthracen-9-ylcarbonyl)piperidin-4-ol
To a solution of 9,10-dihydro-9,10-methano-9-anthracenecarboxylic acid (24.1 mmol) in toluene (70 mL) was added thionyl chloride (2.28 mL, 31.3 mmol, 1.3 eq). The reaction was heated to reflux monitoring gas evolution with a mineral oil bubbler. The system reached a steady state within 40 min at which time it was slightly cooled and 4-hydroxypiperidine (6.08 g, 60.3 mmol, 2.5 eq) was added portionwise. A significant amount of heat is evolved and the reaction became dark. The suspension was heated to reflux for 2 h, cooled to room temperature and stirred for 18 h. The reaction was diluted with ethyl acetate (200 mL) and washed with 3 N HCl (2 x 100 mL), 2.5 N NaOH (2 x 100 mL) and saturated brine (200 mL). The organic phase was dried over anhydrous magnesium sulfate, filtered, and reduced to an oil. The procedure yielded the title compound in quantitative yield as a viscous oil. TLC analysis (R_{f} 0.54, 10 % methanol in CHCl₃). MS (Cl, CH4) m/z 320 (M+1, 100), 348 (M+29,22), 302 (16)
c. 1-(9,10-Dihydro-9,10-methanoanthracen-9-ylmethyl)piperidin-4-ol
To a cooled suspension (0° C) of-(9,10-dihydro-9,10-methanoanthrancen-9-ylcarbonyl)piperidin-4-ol (described above)(19.6 mmol) in diethyl ether (200mL) under nitrogen was added lithium aluminum hydride (1.49 g, 39.2 mmol, 8 eq of hydride) in portions. The suspension was stirred at 0° C for 30 min and warmed to room temperature. After 18 h at room temperature, the excess reagent was carefully quenched with the following in sequence: water (1.5 mL), 2.5 N NaOH (1.5 mL) and additional water (4.5 mL). The suspension was stirred vigorously until the aluminum salts became a granular white solid. The suspension was diluted with ethyl acetate (100 mL), dried with a small amount of anhydrous magnesium sulfate and filtered. The filter cake was rinsed thoroughly with ethyl acetate. The solvent was removed to yield the title compound as a white solid in 88% yield. TLC analysis (R_{f} 0.54, 10% methanol in CHCl₃) MS (Cl, CH4) m/z 306 (M+1, 100), 334 (M+29,14), 288 (62), 114 (8)
d. 1-(9,10-Dihydro-9,10-methanoanthracen-9-ylmethyl)-4-piperidinone
To a cooled solution (-78° C) of oxalyl chloride (3.06 mL, 35.1 mmol, 2 eq) in methylene chloride (100 mL) under nitrogen was added distilled dimethylsulfoxide (5.00 mL, 70.2 mmol, 4 eq). After 10 min 1-(9,10-dihydro-9,10-methanoanthrancen-9-ylmethyl) piperidin-4-ol (described above) (17.5 mmol) was added as a methylene chloride solution (10 mL). The reaction was stirred at -78° C for 30 min prior to the addition of triethylamine (19.6 mL, 140 mmol, 8 eq). The cooling bath was removed and the reaction warmed to room temperature over 1.5 h. The reaction mixture was poured into 2.5 N NaOH (100 mL) and the aqueous phase extracted with methylene chloride ( 3x 100 mL). Combined organic extracts were dried over anhydrous magnesium sulfate, filtered, and reduced to an oil. The crude reaction mixture was purified by flash chromatography over silica gel (400 mL, eluent:20% ethyl acetate in hexane) to yield the title compound in 80% yield as a white solid. TLC analysis (R_{f} 0.31, 2% methanol in methylene chloride). MS (Cl, CH4) m/z 304 (M+1, 100), 332 (M+29,21)
e. 1-(9,10-Dihydro-9,10-methanoanthracen-9-ylmethyl)-4-(2-fluoro-3-pyridy 1)-piperidin-4-ol
To a cooled solution (-72° C) of distilled diisopropylamine (7.86 mL, 56.1 mmol, 1.3 eq) in THF/hexane (57 mL/37 mL) under nitrogen was added n-butyllithium (2.5 M in hexane, 23.8 mL, 59.4 mmol, 1.4 eq). The resulting solution was warmed to -20° C to assure deprotonation and then recooled to -72° C. A THF solution (13 mL) of 2-fluoropyridine (4.50 mL, 53.5 mmol, 1.25 eq) was then added dropwise resulting in a yellow precipitate. The deprotonation reaction was warmed to -50° C for 45 min and briefly allowed to reach -30° C before being recooled to -72° C. To this solution was added a mixture of 1-(9,10-dihydro-9,10-methanoanthracen-9-ylmethyl)-4-piperidone (described in 1d above)(13.0 g, 42.9 mmol) and lithium bromide (7.45 g, 85.8 mmol, 2 eq) in THF (48 mL) in a dropwise fashion. In the course of the addition, the yellow precipitate dissolved. The reaction was warmed to -20° C over 1.5 h and quenched with acetic acid (10 mL). The solution was diluted with water (400 mL), basified with 2.5 N NaOH and extracted with ethyl acetate (3 x 300 mL). The combined organic extracts were dried over anhydrous sodium sulfate, filtered and reduced to a solid. The product was purified by recrystallization from ethyl acetate (3 crops) to yield 13.3 g (77%) of the title compound as a white solid. TLC analysis (R_{f} 0.22, 30% ethyl acetate in hexane). 1H NMR (CDCl₃, 300 mHz) 8.09 (d, J=8.2 Hz, 1H), 7.91 (dd, J=8.2, 9.4 Hz, 1H), 7.21 (m, 5H), 6.94 (m, 4H), 4.27 (s, 1H), 3.48 (s, 2H), 2.91 (m, 2H), 2.74 (m, 2H), 2.62 (s, 2H), 2.26 (m, 2H), 1.78 (m, 2H) MS (Cl,CH4) m/z 401 (M+1, 100), 429 (M+29,15), 383 (21) The free base was dissolved in ether and acidified with ethereal HCl. The hydrochloride salt was filtered, washed with fresh ether and dried in vacuo (room temperature, 10 pascal, 18 h) to yield a white solid, mp 188-191° C (dec).

| Analysis for C₂₆H₂₅FN₂O^{·}HCl^{·}0.4H₂O: | | | |
|---|---|---|---|
| Calculated | C, 70.31; | H, 6.08; | N, 6.31 |
| Found | C, 70.65; | H, 6.12; | N, 5.83 |

f. 1-(9,10-Dihydro-9,10-methanoanthracen-9-ylmethyl)-4-(2-ethylthio-3-pyr idyl)piperidin-4-ol
To a solution of the 1-(9,10-dihydro-9,10-methanoanthracen-9 -ylmethyl)-4-(2-fluoro-3-pyridyl)piperidin-4-ol (described in e. above) (2.00 g, 5.00 mmol, 1 eq) in THF (50 mL) under nitrogen was added the sodium salt of ethanethiol (0.90 g, 10.7 mmol, 2.2 eq). The thiolate salt was prepared from ethanethiol and sodium hydride under standard conditions. The reaction was heated to reflux for 18 h and quenched by pouring into water (100 mL). The aqueous phase was extracted with diethyl ether (2 x 100 mL), dried over anhydrous sodium sulfate, filtered, and reduced to an oil. The reaction product was purified by flash chromatography over silica gel (200 mL, eluent: 50% ether in hexane) to yield 2.00 g (90%) of the title compound. TLC analysis (R_{f} 0.29, 50% ether in hexane).
1HNMR (CDCl₃, 250 MHz) 8.35 (dd, J=1.6, 4.7 Hz, 1H), 7.58 (dd, J=1.7, 7.7 Hz, 1H), 7.22 (m, 4H), 6.95 (m, 5H), 4.27 (s, 1H), 3.58 (s, 1H), 3.48 (s, 2H), 3.28 (q, J=7.3 Hz, 2H), 2.89 (m, 2H), 2.80 (ddd, J=9.3, 14.9, 10.7 Hz, 2H) 2.62 (d, J=1.5 Hz, 2H), 2.12 (m, 4H), 1.35 (t, J=7.2 Hz, 3H) MS (Cl, CH4) m/z 443 (M+1, 100), 471 (M+29,16), 425 (25) The free base was dissolved in ether and acidified with ethereal HCl. The hydrochloride salt was filtered, rinsed with fresh ether and dried in vacuo (room temperature, 10 pascal, 18 h) to yield a white solid, mp 176-179 (dec).

| Analysis for C₂₈H₃₀N₂OS^{·}2HCl^{·}0.5H₂O: | | | |
|---|---|---|---|
| Calculated | C, 64.11; | H, 6.34; | N, 5.34 |
| Found | C, 64.05; | H, 6.32; | N, 5.26 |

### Example 2

### 1-(9,10-Dihydro-9,10-methanoanthracen-9-ylmethyl)-4-(2-ethylthio -3-pyridyl)piperidin-4-ol

### Reduction Process for Producing Sulfides to Recycle in Example 1

Zinc dust (1.72 g, 26.2 mmol) was transferred under nitrogen to a dry 500 mL roundbottom flask equipped with a nitrogen inlet, addition funnel, teflon coated magnetic stirbar and thermocouple. Distilled tetrahydrofuran (200 mL, from sodium/benzophenone ketyl) was added to the reaction vessel via cannula and the resulting suspension cooled to 0° C with an ice bath. Titanium tetrachloride (1.44 mL, 13.1 mmol, recently distilled) was added to the vortex of the rapidly stirred zinc suspension to reduce splattering. The initially formed titanium tetrachloride-tetrahydrofuran complex (yellow) quickly dissipates as reaction with the zinc takes place. After stirring for 10 minutes, a tetrahydrofuran solution (50 mL) of the substrate, 1-(9,10-dihydro-9,10-methanoanthracen-9-ylmethyl)-4-(2-ethylsulfinyl-3 -pyridyl)piperidin-4-ol (1.50 g, 3.27 mmol) was added dropwise to the gray/blue titanium (II) suspension. Reaction was instantaneous with the formation of a titanium (III) suspension (purple/black). The cooling bath was removed, and the reaction was maintained at room temperature for two hours. The reaction was quenched by the addition of water (100 mL) and aqueous sodium hydroxide (2.5 N, 100 mL). The aqueous phase was extracted with ethyl acetate (2 X 250 mL). Combined organic extracts were dried over anhydrous sodium sulfate, filtered and reduced to an oil. TLC analysis (Rf 0.29, 50% ether in hexane) indicated the presence of product only. The reaction product was purified by dissolving the residue in hot toluene (10 mL) and diluting it with hot hexane (20 mL). After brief heating to reflux, this solution was filtered and cooled to room temperature. After the sulfide crystallization appears to have peaked, the recrystallization system was cooled to 0° C. After several hours the solvent was removed and the solid was dried on high vacuum (room temperature, 10 pascal, 1 h). The procedure yielded 1.25 g (86.4%) of the title compound as a yellow solid which appeared analytically pure by NMR. 1H NMR (CDCl3, 250 MHz) 8.35 (dd, J=1.6, 4.7 Hz, 1H), 7.58 (dd, J=1.7, 7.7 Hz, 1H), 7.22 (m, 4H), 6.95 (m 5H), 4.27 (s, 1H), 3.58 (s, 1H), 3.48 (s, 2H), 3.28 (q, J=7.3 Hz, 2H), 2.89 (m, 2H), 2.80 (ddd, J=9.3, 14.9, 10.7 Hz, 2H), 2.62 (d, J=1.5 Hz, 2H), 2.12 (m, 4H), 1.35 (t, J=7.2 Hz, 3H)
MS (CI, CH4) m/z 443 (M+1,100), 471 (M+29,16), 425 (25)

The following example is a preferred procedure for preparing the title compound.

### Example 3

### (-)-1-(9,10-Dihydro-9,10-methanoanthracen-9-ylmethyl)-4-(2-ethyl sulfinyl-3-pyridyl)piperidin-4-ol

To a cooled solution (-16° C) of diethyl D-tartrate (2.10 moles) in undried toluene under nitrogen was added titantium tetraisopropoxide (1.06 moles); 1-(9,10-dihydro-9,10-methanoanthracen -9-ylmethyl)-4-(2-ethylthio-3-pyridyl)piperidin-4-ol (1.0 mole) and t-butyl hydroperoxide (1.18 moles). The reaction was allowed to proceed for 48 hours at which point 2N NaOH solution was added; the solid TiO₂ was filtered off and solvent evaporate to yield the title preferred enantiomer in an 8:1 ratio (65-70% yield).
Recrystallization in MeCN yielded pure (-) enantiomer in 72% yield; mp 185-186 with a specific rotation of [a] - 106° (c = 1.0 in MeOH).

The starting -2-(ethylthio-3-pyridyl)-piperidin-4-ol compound was advantageously prepared as follows:
a. 9-formyl-9,10-dihydro-9,10-methanoanthracene.
   The tricyclic aldehyde shown as 14 in Scheme 1 (X and Y = H) was reacted with vinyl acetate (7.17 eq) in a sealed tube at 180°C (or obtained commercially) to form the ethanoanthracene aldehyde shown as 16 is Scheme 1. Jones oxidation (CrO₃/H₂SO₄) in H₂O/acetone (1.96/7.7 vol) at 20-25°C gave the corresponding 9-carboxylic acid ethanoanthracene shown as 18. Reaction of 18 (X and Y = H) with diphenylphosphoryl azide (6.85 eq) and triethylamine (NEt₃, 1.2 eq) in methylisobutylketone (MIBK) at 90-95°C yielded, via the corresponding intermediate azide and isocyanate, after treatment with 100 T_{w} NaOH (10 eq) in MIBK, a 66% yield of the primary amine (isolated as the HC 1 salt from MIBK) depicted as 20 in Scheme 1. Ring contraction initiated by diazotization of the primary amine using NaNO₂/HOAc (1.5 eq/25 eq) in H₂O gave the starting methanoaldehyde in 90% yield after neutralization with KOH and filtration. Alternatively, treatment of the intermediate isocyanate after treatment with the diphenylphosphoryl azide with acetic acid (0.5 vol); 1.18 mHCl (0.5 vol) in toluene yields the primary amine 20 (X and Y = H) in 72% yield and subsequent ring contraction via a carbocation intermediate using the above diazotization reagents gives the methanoaldehyde in 90% yield.
b. 1-(9,10-dihydro-9,10-methanoanthracen-9-ylmethyl)-4-piperidone.
   The methanoaldehyde produced in step a. above (1.0mole) was reacted with piperidine-4-ethylketal (1.5 mole); 8M BH₃ · py (0.45 mole) in MeOH at 25 °C for four hours to form the ketal protected 1-(9,10-dihydro-9,10-methanoanthracen-9-ylmethyl)4-piperidone which was deprotected by sequential treatment with (1) conc. HCl (reflux, 2 hours, 2X) and (2) 47% NaOH solution at 85° C to pH > 8 until the product (the 4-pieridone title compound) precipitates in 79% yield.
c. 1-(9,10-Dihydro-9,10-methanoanthracen-9-ylmethyl)-4-(2-fluoro-3-pyridyl)-piperidin-4-ol.
   LDA (1.1 moles) was produced from nBuLi and diisopropyl amine and reacted with 2-flouropyridine (1.1 moles) at -70 °C to form the pyridyl anion which was then reacted with the 4-piperidone produced in step b. to form the title compound. The reaction was quenched with AcOH, partitioned between H₂O/toluene and crystallized from toluene in 66% yield.
d. 1-(9,10-Dihydro-9,10-methanoanthracen-9-ylmethyl)-4-(2-ethylthio -3-pyridyl)-piperidin-4-ol.
   To a solution of the title compound described in c. above (1.0 mole) was added 2 moles of EtSH, 4N NaOH (2.05 moles) in 1-methylpyrollidone at 90 °C for seven hours. After partitioning between H₂O/toluene, the toluene was evaporated and the title compound was crystallized from isopropanol in 79% yield. This compound was then utilized to form crystalline (-)-1-(9,10-Dihydro-9,10-methanoanthracen-9-ylmethyl)-4-(2-ethylsulfin yl-3-pyridyl)-piperidin-4-ol.

### Example 4

The following illustrate representative pharmaceutical dosage forms containing a compound of formula I selected from, for example, a pure form or substantially pure form of (-)-1-(9,10-Dihydro-9,10-methanoanthracen-9-ylmethyl)-4-(2-ethylsulfin yl-3-pyridyl)piperidin-4-ol. A tablet preparation may be composed of the above compound(s)(50.0 mg) for therapeutic or prophylactic use in humans and may also contain pharmaceutically acceptable excipients such as Mannitol, USP (223.75 mg); croscarmellose sodium (6.0 mg); maize starch (15.0 mg); hydroxypropylmethylcellulose (2.25 mg) and magnesium stearate (3.0 mg). A capsule formulation containing the above active ingredient (10 mg) for oral administration may contain pharmaceutically acceptable excipients such as mannitol, USP (488.5 mg); croscarmellose sodium (15.0 mg) and magnesium stearate (1.5 mg). A suitable oil formulation may also be prepared using, for example, the free base of the above compound and a 25% glycerine in water mixture. This can be used, for example, in gel caps or other suitable delivery means. The above formulations may be prepared by conventional means or methods known in the art. The above examples are considered to be non-limiting and other known and/or conventional pharmaceutically acceptable excipients or diluents may be added to the active ingredient(s) to form a pharmaceutical composition which is useful in the treatment of neurological disorders including treatment of psychoses in patients in need of treatment thereof. The present invention therefore relates to a pharmaceutical composition comprising an effective amount of the active compounds as described herein in admixture with known pharmaceutically acceptable carriers. The present invention further relates to a method for antagonizing the effects of dopamine receptor (D1 and D2) agonists or serotonin 5HT2 agonists by antagonizing the respective receptor sites comprising administering to a patient in need of treatment thereof, an antagonist amount of the compounds or compositions as described herein. The invention therefore also relates to a method of treating neurological disorders affected, acerbated or caused by excessive activation of D1, D2 or 5HT2 receptors comprising administering an antagonistic amount of a compound according to formula I.

The compound (-)-1-(9,10-dihydro-9,10-methanoanthracen-9-yl-methyl)-4-(2-ethylsulfinyl)-3 pryidyl)piperidin-4-ol has good equilibrium solubility in aqueous and non-aqueous media and retains chemical and stereochemical stability in solution. The compound as the free amine is also advantageously non-hygroscopic.

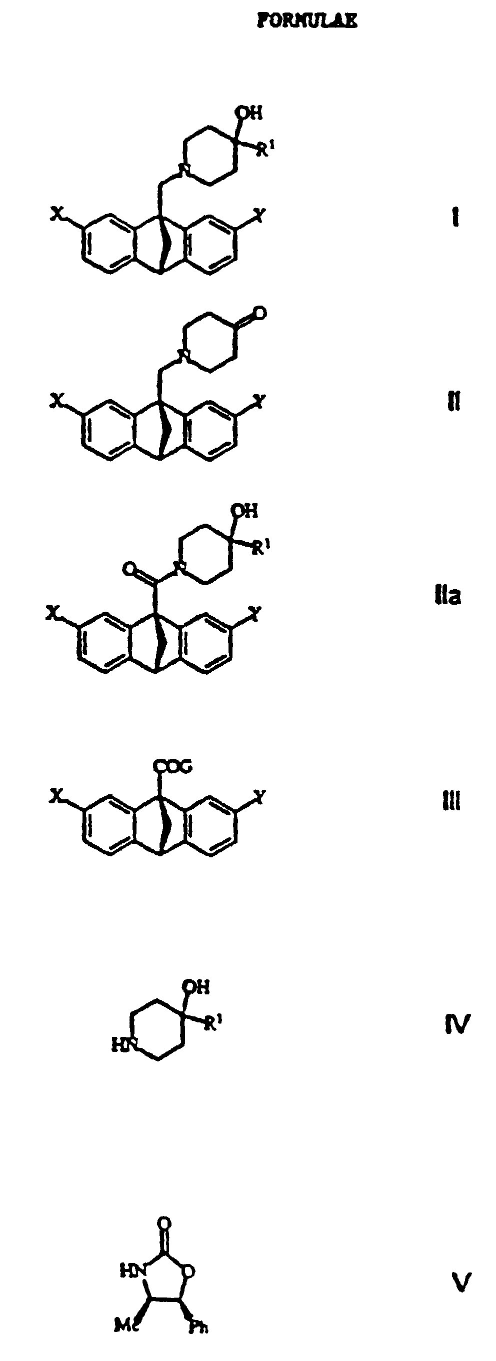

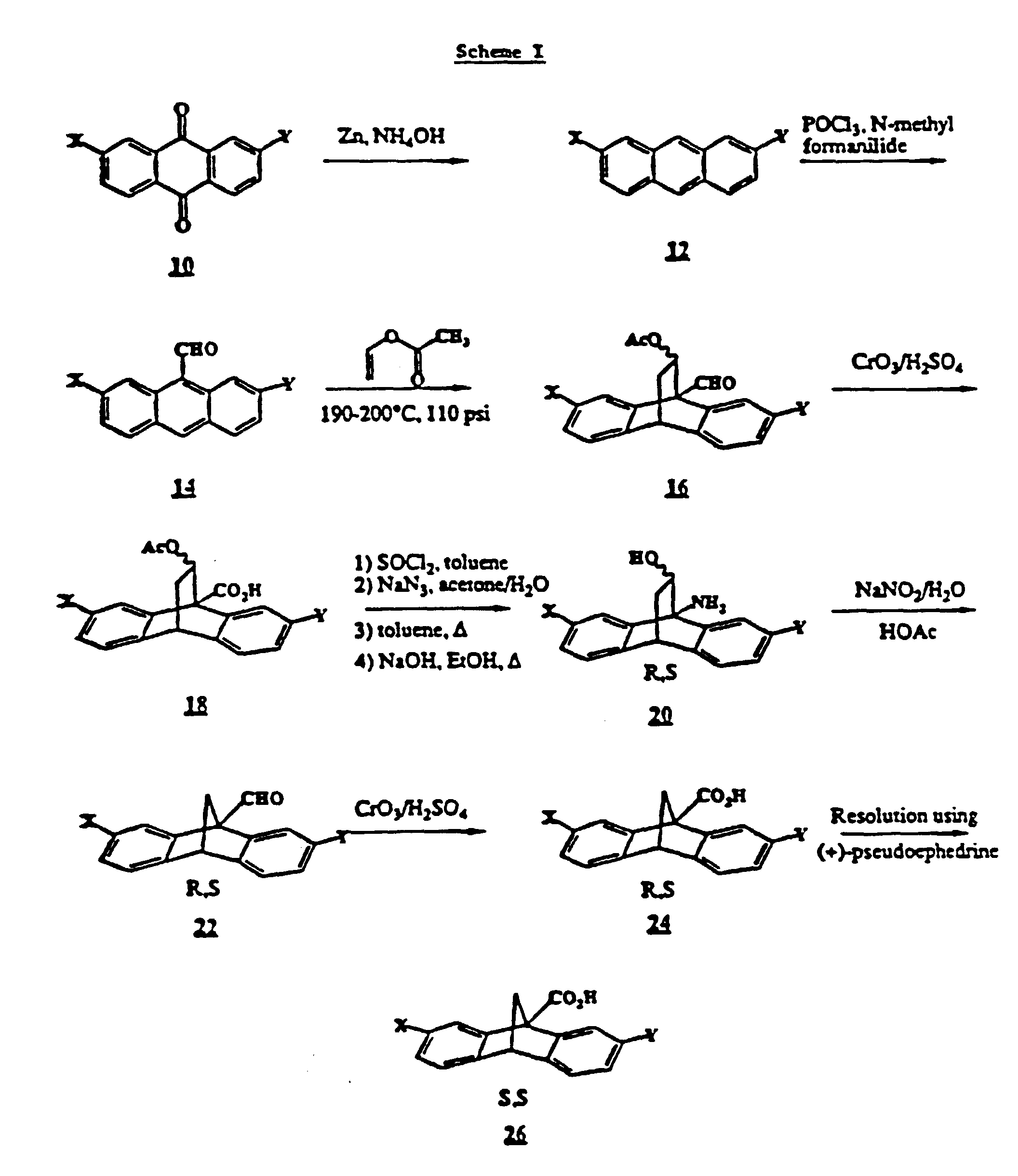

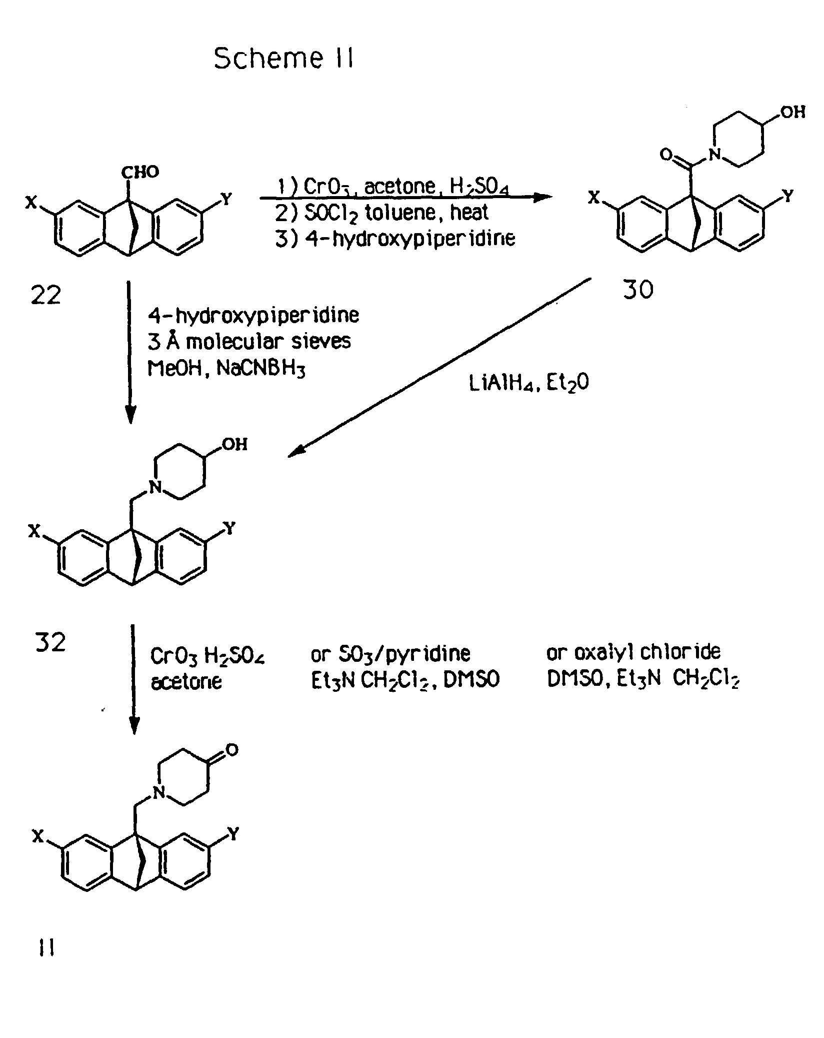

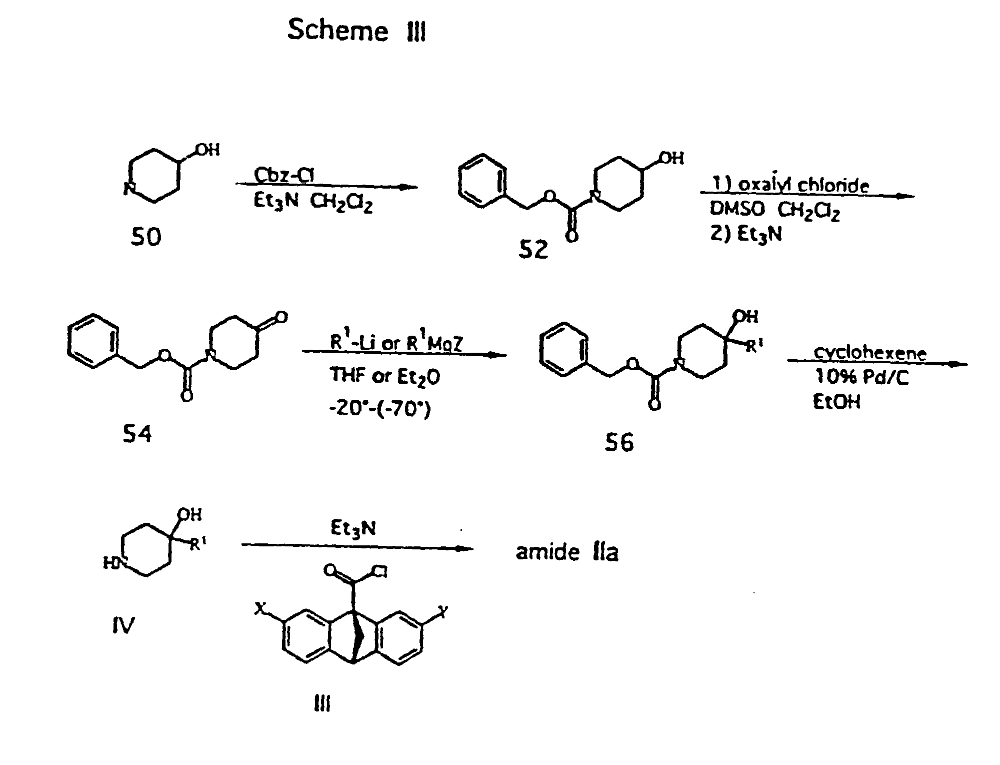

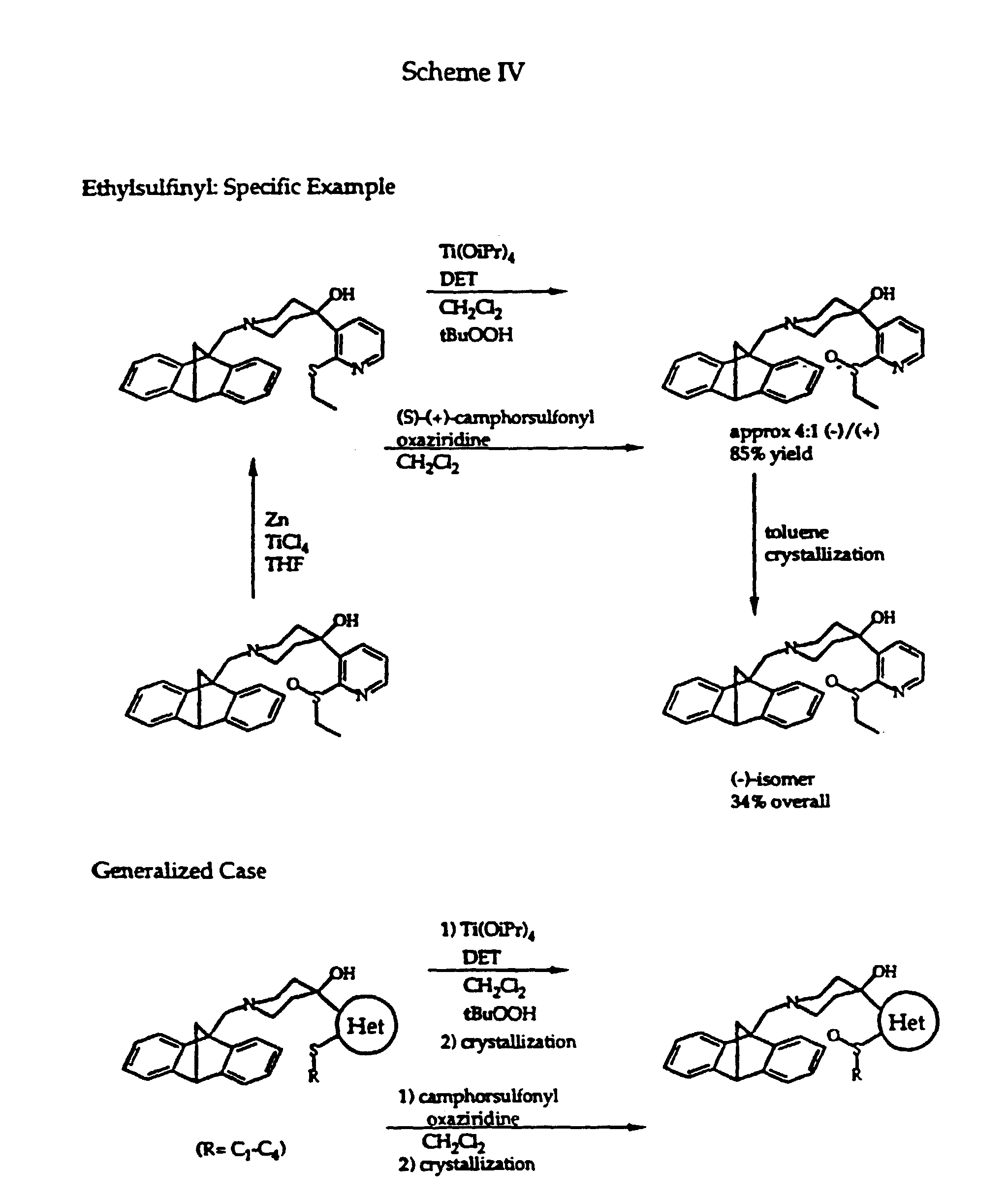

## Claims

1. An optically active compound of formula I: 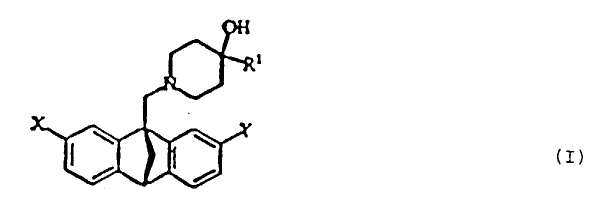 wherein:
X and Y are independently selected from hydrogen, halogen and C1-6alkoxy;
R1 is selected from:
Five- and six- membered heteroaryl rings containing from 1-3 heteroatoms selected from nitrogen, oxygen, and sulfur, and benz derivatives thereof, which may bear 1-2 substituents selected from C1-6 alkyl, hydroxy, C1-6 alkoxy which may bear a trifluoromethyl group, C1-6 alkoxycarbonyl, C1-6 hydroxyalkyl, benzyloxy, halo, C1-3 alkylaminocarbonylC1-3alkyl, aminocarbonyl having the formula: CONR^{c}R^{d} wherein R^{c} and R^{d} are independently selected from hydrogen, 2-pyrrolidinyl, and C₁₋₆ alkyl or wherein R^{c} and R^{d} together with the nitrogen atom to which each is attached form a 5- or 6- membered heterocyclic ring in which said nitrogen is the only heteroatom, R^{e}S(O)ₙ, R^{f}NH, and R^{g}S wherein R^{e} and R^{f} are independently selected from hydrogen and C₁₋₆ alkyl and n is 0, 1 or 2 and R^{g} is selected from C₁₋₃ alkylcarbonylaminophenyl and di(C₁₋₃)alkylamino(C₁₋₆)alkyl, wherein at least one of the substituents on the five- and/or six-membered heteroaryl ring is a chiral sulfoxide of the formula C₁₋₄alkylSO, and pharmaceutically acceptable salts thereof.

2. A compound according to claim 1 wherein X and Y are H and R¹ is selected from 2-ethylsulfinyl-3-pyridyl and pharmaceutically acceptable salts thereof.

3. A compound according to claim 1 wherein the compound of formula I is (-)-1-(9,10-dihydro-9,10-methanoanthracen-9-ylmethyl)-4-(2-ethylsulfinyl-3-pyridyl)piperidin-4-ol characterized in that said compound is crystalline and pharmaceutically acceptable salts thereof.

4. A compound according to claim 3 characterized in that said crystalline compound has a melting point of 185-186°C and a specific rotation of [a] -106° (c = 1.0 in MeOH) and is non-hygroscopic.

5. A crystalline compound according to claim 1 wherein the compound of formula I is (-)-1-(9,10-dihydro-9,10-methanoanthracen-9-yl(methyl)-4-(2-ethylsulfinyl)-3-pyridyl)piperidin-4-ol characterized in that it has good equilibrium solubility in aqueous and non-aqueous media and retains chemical and stereochemical stability in solution.

6. A pharmaceutical composition comprising a compound according to claim 1 and a pharmaceutically acceptable carrier or diluent.

7. A pharmaceutical composition comprising a crystalline compound of (-)-1-(9,10-Dihydro-9,10-methanoanthracen-9-ylmethyl)-4-(2-ethylsulfinyl-3-pyridyl)piperidin-4-ol or a pharmaceutically acceptable salt thereof, and a pharmaceutically acceptable carrier or diluent.

8. A process for preparing a compound of formula I, or a pharmaceutically acceptable salt thereof, as claimed in any one of claims 1 to 5 which comprises oxidising a compound of formula I, in which X and Y are as defined in any one of claims 1 to 5 and R1 is as defined in any one of claims 1 to 5 with the exception that the or each of said groups of formula-C₁₋₄alkylSO is of formula C₁₋₄alkylS, via a chiral oxidation process so as to enantioselectively oxidize the said group of formula C₁₋₄alkylS to a chiral sulfoxide of the formula C₁₋₄alkylSO, thereby producing a compound of formula I as defined in any one of claims 1 to 5; and where a pharmaceutically acceptable salt is required, treating the compound of formula I with an acid which forms a physiologically acceptable anion or a base which affords a physiologically acceptable cation.

9. A process for preparing a compound of formula I, or a pharmaceutically-acceptable salt thereof, as claimed in any one of claims 1 to 5 which comprises:
(a) treating a piperidone having formula II: 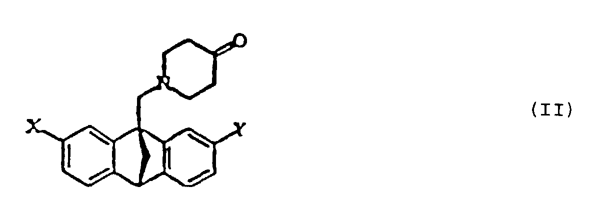 with a corresponding compound of formula R¹Li in an aprotic solvent;
(b) treating a corresponding amide of formula IIa: 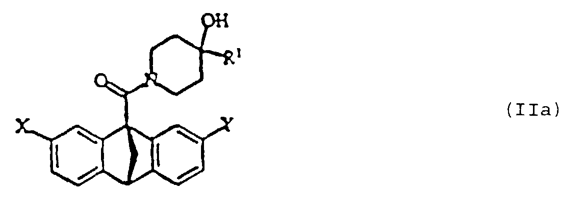 with a suitable reducing agent;
(c) treating an aldehyde of formula III: 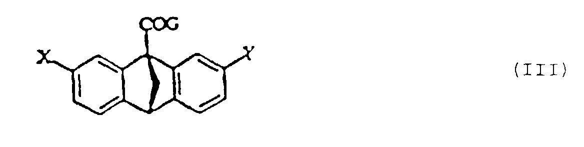 (G is hydrogen) with a corresponding piperidine of formula IV in the presence of a reducing agent; or
(d) for a compound of formula I wherein the value of C₁₋₆ alkoxy for either X or Y is desired, treating a corresponding compound of formula I wherein X or Y is hydroxy with a desired base and alkylhalide to form the alkoxy compound(s);
and whereafter treating the compound obtained in (a), (b), (c) or (d) with an enantioselective oxidsing agent;
and where a pharmaceutically acceptable salt is required, treating the compound of formula I with an acid which forms a physiologically acceptable anion or a base which affords a physiologically acceptable cation.

10. A process as claimed in claim 8 or 9 wherein the oxidising agent is selected from a titanium/tartarate/peroxide mixture and a chiral oxaziridine.

11. A process for producing crystalline (-)-1-(9,10-Dihydro-9,10-methanoanthracen-9-ylmethyl)-4-(2-ethylsulfinyl-3-pyridyl)piperidin-4-ol which comprises treating 1-(9,10-dihydro-9,10-methanoanthracen-9-ylmethyl)-4-(2-ethylthio-3-pyridyl)piperidin-4-ol with a cooled solution of diethyl D-tartrate in undried toluene or equivalent solvent; titantium tetraisopropoxide and t-butyl hydroperoxide to form an enantiomerically enriched ethylsulfinyl mixture which is then recrystallized in a suitable solvent such as MeCN or equivalent to form pure (-) enantiomer.

12. The use of a compound of formula I, or a pharmaceutically-acceptable salt thereof, as claimed in any one of claims 1 to 5 for the manufacture of a medicament for the treatment of psychiatric disorders.

13. The use of a compound of formula I, or a pharmaceutically-acceptable salt thereof, as claimed in any one of claims 1 to 5 for the manufacture of a medicament for the treatment of neurological disorders.

14. The crystalline compound (-)-l-(9,10-dihydro-9,10-methanoanthracen-9-ylmethyl)-4-(2-ethylsulfinyl-3-pyridyl)piperidin-4-ol, or a pharmaceutically acceptable salt thereof, for use in medicine.

15. Use of the crystalline compound (-)-1-(9,10-dihydro-9,10-methanoanthracen-9-ylmethyl)-4-(2-ethylsulfinyl-3-pyridyl)piperidin-4-ol, or a pharmaceutically acceptable salt thereof, for the manufacture of a medicament for the treatment of psychiatric disorders in humans in need of treatment thereof.

## Patentansprüche

1. Optisch aktive Verbindung der Formel I 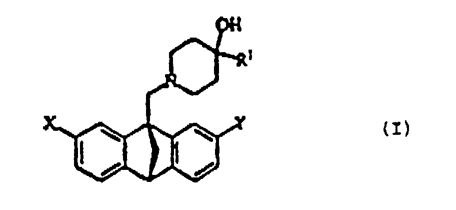 worin:
X und Y unabhängig voneinander aus Wasserstoff, Halogen und C₁₋₆-Alkoxy ausgewählt sind;
R¹ ausgewählt ist aus:
fünf- und sechsgliedrigen Heteroarylringen, die 1-3 Heteroatome enthalten, ausgewählt aus Stickstoff, Sauerstoff und Schwefel, und ihren Benzderivaten, die 1-2 Substituenten tragen können, ausgewählt aus C₁₋₆-Alkyl, Hydroxy, C₁₋₆-Alkoxy, das eine Trifluormethylgruppe tragen kann, C₁₋₆-Alkoxycarbonyl, C₁₋₆-Hydroxyalkyl, Benzyloxy, Halogen, C₁₋₃-Alkylaminocarbonyl-C₁₋₃-alkyl, Aminocarbonyl der Formel: CONR^{c}R^{d}, worin R^{c} und R^{d} Unabhängig voneinander ausgewählt sind aus Wasserstoff, 2-Pyrrolidinyl und C₁₋₆-Alkyl oder worin R^{c} und R^{d} zusammen mit dem Stickstoffatom, an das sie jeweils gebunden sind, einen 5- oder 6-gliedrigen heterozyklischen Ring bilden, in dem der Stickstoff das einzige Heteroatom ist, R^{e}S(O)ₙ, R^{f}NH und R^{g}S, worin R^{e} und R^{f} unabhängig voneinander ausgewählt sind aus Wasserstoff und C₁₋₆-Alkyl, und n 0, 1 oder 2 ist und R^{g} ausgewählt ist aus C₁₋₃-Alkylcarbonylaminophenyl und Di(C₁₋₃)alkylamino(C₁₋₆)alkyl, wobei mindestens einer der Substituenten am fünf- und/oder sechsgliedrigen Heteroarylring ein chirales Sulfoxid der Formel C₁₋₄-AlkylSO ist; und pharmazeutisch verträgliche Salze davon.

2. Verbindung nach Anspruch 1, worin X und Y ausgewählt sind aus H, und R¹ ausgewählt ist aus 2-Ethylsulfinyl-3-pyridyl, und pharmazeutisch verträgliche Salze davon.

3. Verbindung nach Anspruch 1, wobei die Verbindung der Formel I (-)-1-(9,10-Dihydro-9,10-methanoanthracen-9-ylmethyl)-4-(2-ethylsulfinyl-3-pyridyl)piperidin-4-ol ist, dadurch gekennzeichnet, daß die Verbindung kristallin ist, und pharmazeutisch verträgliche Salze davon.

4. Verbindung nach Anspruch 3, dadurch gekennzeichnet, daß die kristalline Verbindung einen Schmelzpunkt von 185-186°C und eine spezifische Drehung von [α] -106° (c = 1,0 in MeOH) hat und nicht hygroskopisch ist.

5. Kristalline Verbindung nach Anspruch 1, wobei die Verbindung der Formel I (-)-1-(9,10-Dihydro-9,10-methanoanthracen-9-ylmethyl)-4-(2-ethylsulfinyl-3-pyridyl)piperidin-4-ol ist, dadurch gekennzeichnet, daß sie eine gute Gleichgewichtslöslichkeit in wäßrigen und nicht-wäßrigen Medien hat und in Lösung chemische und stereochemische Stabilität behält.

6. Arzneimittel, umfassend eine Verbindung nach Anspruch 1 und ein(en) pharmazeutisch verträgliche(n/s) Träger oder Verdünnungsmittel.

7. Arzneimittel, umfassend eine kristalline Verbindung von (-)-1-(9,10-Dihydro-9,10-methanoanthracen-9-ylmethyl)-4-(2-ethylsulfinyl-3-pyridyl)piperidin-4-ol oder ein pharmazeutisch verträgliches Salz davon, und ein(en) pharmazeutisch verträgliche(n/s) Träger oder Verdünnungsmittel.

8. Verfahren zum Herstellen einer Verbindung der Formel I oder eines pharmazeutisch verträglichen Salzes davon nach einem der Ansprüche 1 bis 5, umfassend das Oxidieren einer Verbindung der Formel I, wobei X und Y die in einem der Ansprüche 1 bis 5 definierte Bedeutung haben und R¹ die in einem der Ansprüche 1 bis 5 definierte Bedeutung hat, mit der Ausnahme, daß die oder jeder der Reste der Formel C₁₋₄-AlkylSO die Formel C₁₋₄-AlkylS hat, über ein chirales Oxidationsverfahren, so daß der Rest der Formel C₁₋₄-Alkyls zu einem chiralen Sulfoxid der Formel C₁₋₄-AlkylSO enantioselektiv oxidiert wird, wodurch eine Verbindung der Formel I nach einem der Ansprüche 1 bis 5 hergestellt wird; und wenn ein pharmazeutisch verträgliches Salz gewünscht ist, das Behandeln der Verbindung der Formel I mit einer Säure, die ein physiologisch verträgliches Anion bildet, oder einer Base, die ein physiologisch verträgliches Kation liefert.

9. Verfahren zum Herstellen einer Verbindung der Formel I oder eines pharmazeutisch verträglichen Salzes davon nach einem der Ansprüche 1 bis 5, umfassend:
(a) das Behandeln eines Piperidons der Formel II 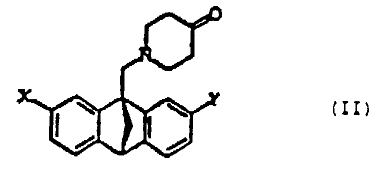 mit einer entsprechenden Verbindung der Formel R¹Li in einem aprotischen Lösungsmittel;
(b) das Behandeln eines entsprechenden Amides der Formel IIa 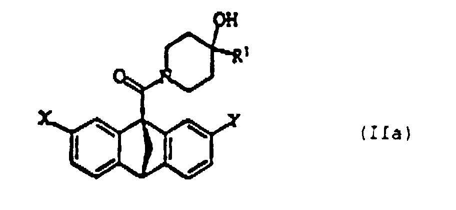 mit einem geeigneten Reduktionsmittel;
(c) das Behandeln eines Aldehydes der Formel III 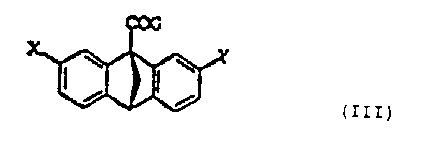 (G is Wasserstoff) mit einem entsprechenden Piperidin der Formel IV in Gegenwart eines Reduktionsmittels; oder
(d) für eine Verbindung der Formel I, wobei die Bedeutung C₁₋₆-Alkoxy für entweder X oder Y gewünscht ist, Behandeln einer entsprechenden Verbindung der Formel I, worin X oder Y Hydroxy ist, mit einer gewünschten Base und Alkylhalogenid, so daß die Alkoxyverbindung(en) hergestellt wird/werden;
und anschließend das Behandeln der in (a), (b), (c) oder (d) gewonnenen Verbindung mit einem enantioselektiven Oxidationsmittel;
und wenn ein pharmazeutisch verträgliches Salz benötigt wird, das Behandeln der Verbindung der Formel I mit einer Säure, die ein physiologisch verträgliches Anion bildet, oder einer Base, die ein physiologisch verträgliches Kation liefert.

10. Verfahren nach Anspruch 8 oder 9, wobei das Oxidationsmittel ausgewählt ist aus einem Titan/Tartrat/Peroxid-Gemisch und einem chiralen Oxaziridin.

11. Verfahren zur Herstellung von kristallinem (-)-1-(9,10-Dihydro-9,10-methanoanthracen-9-ylmethyl)-4-(2-ethylsulfinyl-3-pyridyl)piperidin-4-ol, umfassend das Behandeln von 1-(9,10-Dihydro-9,10-methanoanthracen-9-ylmethyl)-4-(2-ethylthio-3-pyridyl)-piperidin-4-ol mit einer gekühlten Lösung von Diethyl-D-tartat in nicht getrocknetem Toluol oder einem äquivalenten Lösungsmittel, Titantetraisopropoxid und tert.-Butylhydroperoxid, so daß ein enantiomer angereichertes Ethylsulfinylgemisch hergestellt wird, das dann in einem geeigneten Lösungsmittel, wie MeCN oder einem Äquivalent, umkristallisiert wird, so daß das reine (-)-Enantiomer hergestellt wird.

12. Verwendung einer Verbindung der Formel I oder eines pharmazeutisch verträglichen Salzes davon nach einem der Ansprüche 1 bis 5 zur Herstellung eines Medikamentes zur Behandlung von psychiatrischen Strörungen.

13. Verwendung einer Verbindung der Formel I oder eines pharmazeutisch verträglichen Salzes davon nach einem der Ansprüche 1 bis 5 zur Herstellung eines Medikamentes zur Behandlung von neurologischen Strörungen.

14. Kristalline Verbindung (-)-1-(9,10-Dihydro-9,10-methanoanthracen-9-ylmethyl)-4-(2-ethylsulfinyl-3-pyridyl)piperidin-4-ol oder ein pharmazeutisch verträgliches Salz davon zur Verwendung in der Medizin.

15. Verwendung der kristallinen Verbindung (-)-1-(9,10-Dihydro-9,10-methanoanthracen-9-ylmethyl)-4-(2-ethylsulfinyl-3-pyridyl)piperidin-4-ol oder eines pharmazeutisch verträglichen Salzes davon zur Herstellung eines Medikamentes zur Behandlung von psychiatrischen Störungen bei Menschen, die diesbezüglich eine Behandlung benötigen.

## Revendications

1. Composé optiquement actif de formule I : 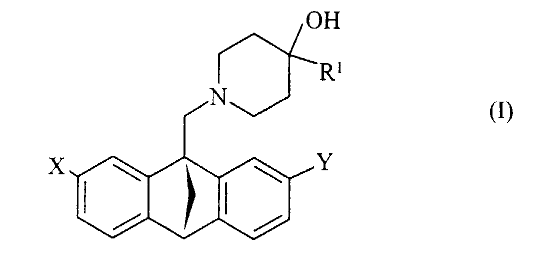 où :
X et Y sont choisis indépendamment parmi hydrogène, halogène et alcoxy en C₁₋₆ ;
R¹ est choisi parmi :
les cycles hétéroaryle à 5 et 6 chaînons contenant de 1 à 3 hétéroatomes choisis parmi l'azote, l'oxygène et le soufre, et leurs dérivés benzo, pouvant porter 1 à 2 substituants choisis parmi alkyle en C₁₋₆, hydroxyle, alcoxy en C₁₋₆ qui peut porter un groupe trifluorométhyle, alcoxy en C₁₋₆-carbonyle, hydroxyalkyle en C₁₋₆, benzyloxy, halogéno, alkyle en C₁₋₃-aminocarbonyl-alkyle en C₁₋₃, aminocarbonyle de formule CONR^{c}R^{d} dans laquelle R^{c} et R^{d} sont choisis indépendamment parmi hydrogène, 2-pyrrolidinyle et alkyle en C₁₋₆ ou dans laquelle R^{c} et R^{d} forment avec l'atome d'azote auquel ils sont liés un cycle hétérocyclique à 5 ou 6 chaînons dans lequel ledit azote est le seul hétéroatome, R^{e}S(O)ₙ, R^{f}NH et R^{g}S où R^{e} et R^{f} sont choisis indépendamment parmi hydrogène et alkyle en C₁₋₆ et n est 0, 1 ou 2 et R^{g} est choisi parmi alkyle en C₁₋₃-carbonylaminophényle et dialkyle en C₁₋₃-aminoalkyle en C₁₋₆ où au moins l'un des substituants sur le cycle hétéroaryle à 5 et/ou 6 chaînons est un sulfoxyde chiral de formule alkyle en C₁₋₄SO, et ses sels pharmaceutiquement acceptables.

2. Composé selon la revendication 1 où X et Y représentent H et R¹ est choisi parmi 2-éthylsulfinyl-3-pyridyle et ses sels pharmaceutiquement acceptables.

3. Composé selon la revendication 1 où le composé de formule I est le (-)-1-(9,10-dihydro-9,10-méthanoanthracén-9-ylméthyl)-4-(2-éthylsulfinyl-3-pyridyl)pipéridin-4-ol caractérisé en ce que ledit composé est cristallin, et ses sels pharmaceutiquement acceptables.

4. Composé selon la revendication 3 caractérisé en ce que ledit composé cristallin a un point de fusion de 185-186°C et un pouvoir rotatoire spécifique [a] = -106° (c = 1,0 dans MeOH) et est non hygroscopique.

5. Composé cristallin selon la revendication 1 où le composé de formule I est le (-)-1-(9,10-dihydro-9,10-méthanoanthracén-9-ylméthyl)-4-(2-éthylsulfinyl-3-pyridyl)pipéridin-4-ol caractérisé en ce qu'il a une bonne solubilité à l'équilibre dans les milieux aqueux et non aqueux et conserve une stabilité chimique et stéréochimique en solution.

6. Composition pharmaceutique comprenant un composé selon la revendication 1 et un support ou diluant pharmaceutiquement acceptable.

7. Composition pharmaceutique comprenant un composé cristallin de (-)-1-(9,10-dihydro-9,10-méthanoanthracén-9-ylméthyl)-4-(2-éthylsulfinyl-3-pyridyl)pipéridin-4-ol, ou un sel pharmaceutiquement acceptable de celui-ci, et un support ou diluant pharmaceutiquement acceptable.

8. Procédé pour préparer un composé de formule I, ou un sel pharmaceutiquement acceptable de celui-ci, selon l'une quelconque des revendications 1 à 5 qui comprend l'oxydation d'un composé de formule I où X et Y sont tels que définis dans l'une quelconque des revendications 1 à 5 et R¹ est tel que défini dans l'une quelconque des revendications 1 à 5 avec l'exception que le ou chacun desdits groupes de formule alkyle en C₁₋₄SO est de formule alkyle en C₁₋₄S, par le biais d'un procédé d'oxydation chirale de manière à oxyder énantiosélectivement ledit groupe de formule alkyle en C₁₋₄S en un sulfoxyde chiral de formule alkyle en C₁₋₄SO, pour produire ainsi un composé de formule I tel que défini dans l'une quelconque des revendications 1 à 5 ;
et lorsqu'un sel pharmaceutiquement acceptable est nécessaire, le traitement du composé de formule I avec un acide qui forme un anion physiologiquement acceptable ou avec une base qui donne un cation physiologiquement acceptable.

9. Procédé pour préparer un composé de formule I, ou un sel pharmaceutiquement acceptable de celui-ci. selon l'une quelconque des revendications 1 à 5 qui comprend :
(a) le traitement d'une pipéridone de formule II : 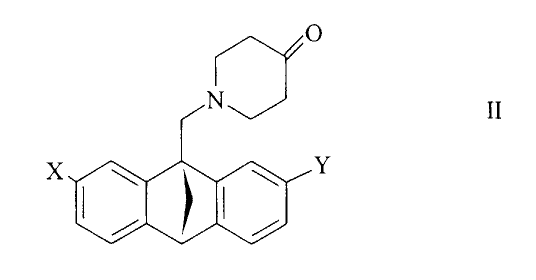 avec un composé correspondant de formule R¹Li dans un solvant aprotique ;
(b) le traitement d'un amide correspondant de formule IIa : 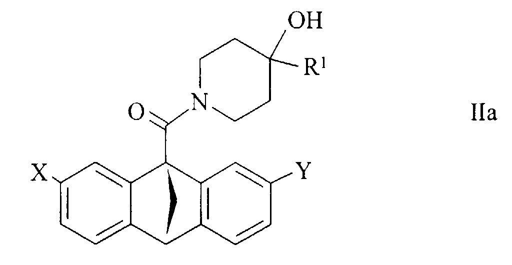 avec un agent réducteur approprié ;
(c) le traitement d'un aldéhyde de formule III : 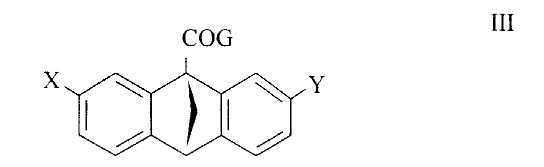 (G est hydrogène) avec une pipéridine correspondante de formule IV en présence d'un agent réducteur ; ou
(d) pour un composé de formule I où la signification de alcoxy en C₁₋₆ est souhaitée pour X ou Y, le traitement d'un composé correspondant de formule I où X ou Y est hydroxyle avec une base voulue et un halogénure d'alkyle pour former le ou les composés alcoxy ;
puis le traitement du composé obtenu en (a), (b), (c) ou (d) avec un agent oxydant énantiosélectif ;
et lorsqu'un sel pharmaceutiquement acceptable est nécessaire, le traitement du composé de formule I avec un acide qui forme un anion physiologiquement acceptable ou avec une base qui donne un cation physiologiquement acceptable.

10. Procédé selon la revendication 8 ou 9 où l'agent oxydant est choisi parmi un mélange titane/tartrate/peroxyde et une oxaziridine chirale.

11. Procédé pour produire le (-)-1-(9,10-dihydro-9,10-méthanoanthracén-9-ylméthyl)-4-(2-éthylsulfinyl-3-pyridyl)pipéridin-4-ol cristallin qui comprend le traitement du 1-(9,10-dihydro-9,10-méthanoanthracén-9-ylméthyl)-4-(2-éthylthio-3-pyridyl)pipéridin-4-ol avec une solution refroidie de D-tartrate de diéthyle dans le toluène non séché ou un solvant équivalent, de tétraisopropylate de titane et d'hydroperoxyde de t-butyle pour former un mélange d'éthylsulfinyle enrichi du point de vue énantiomérique qui est ensuite recristallisé dans un solvant approprié comme MeCN ou équivalent pour former l'énantiomère (-) pur.

12. Utilisation d'un composé de formule I, ou d'un sel pharmaceutiquement acceptable de celui-ci, selon l'une quelconque des revendications I à 5 pour la préparation d'un médicament pour le traitement des troubles psychiatriques.

13. Utilisation d'un composé de formule I, ou d'un sel pharmaceutiquement acceptable de celui-ci, selon l'une quelconque des revendications 1 à 5 pour la préparation d'un médicament pour le traitement des troubles neurologiques.

14. Composé cristallin (-)-1-(9,10-dihydro-9,10-méthanoanthracén-9-ylméthyl)-4-(2-éthylsulfinyl-3-pyridyl)pipéridin-4-ol, ou sel pharmaceutiquement acceptable de celui-ci, destiné à être utilisé en médecine.

15. Utilisation du composé cristallin (-)-1-(9,10-dihydro-9,10-méthanoanthracén-9-ylméthyl)-4-(2-éthylsulfinyl-3-pyridyl)pipéridin-4-ol, ou d'un sel pharmaceutiquement acceptable de celui-ci, pour la préparation d'un médicament pour le traitement de troubles psychiatriques chez des humains nécessitant leur traitement.
